# EUROPEAN PATENT APPLICATION

(11) **EP 4 470 548 A1**
(43) Date of publication of application: **04.12.2024**
(21) Application number: 22923662.5
(22) Date of filing: 29.12.2022
(51) Int. Cl.: A61K 35/741, A61P 1/12, C12N 1/20, C12R 1/01

(54) **USE OF BACTEROIDES FRAGILIS IN IMPROVEMENT AND TREATMENT OF DIARRHEA**

(30) Priority: 25.01.2022 CN 202210089886; 28.10.2022 CN 202211339890
(71) Applicant: Guangzhou Zhiyi Biotechnology Co., Ltd., Guangzhou, Guangdong 510530 (CN)
(72) Inventor: LIU, Yangyang, Guangzhou, Guangdong 510530 (CN); WANG, Ye, Guangzhou, Guangdong 510530 (CN); ZHI, Fachao, Guangzhou, Guangdong 510530 (CN); ZHENG, Lijun, Guangzhou, Guangdong 510530 (CN); WANG, Wei, Guangzhou, Guangdong 510530 (CN); CHANG, Xiujuan, Guangzhou, Guangdong 510530 (CN); LI, Ping, Guangzhou, Guangdong 510530 (CN)
(74) Representative: Gille Hrabal Partnerschaftsgesellschaft mbB Patentanwälte
(86) International application number: PCT/CN2022/143593
(87) International publication number: WO 2023/142877

(57) **Abstract**

Provided is the use of *Bacteroides fragilis* in the improvement and/or treatment of diarrhea. Tests of different mouse diarrhea models prove that *Bacteroides fragilis* ZY-312 with the deposit number CGMCC No.10685 or inactivated *Bacteroides fragilis* ZY-312 has the effect of improving and treating diarrhea. The powder of the inactivated *Bacteroides fragilis* has a good effect of improving infectious diarrhea or non-infectious diarrhea in different concentrations of the formula, and has no side effects on the body.

## Description

### CROSS-REFERENCE TO RELATED APPLICATIONS

**The present invention claims the priority to the prior application with the patent application No.** 202210089886.6 entitled "USE OF BACTEROIDES FRAGILIS IN IMPROVEMENT AND TREATMENT OF DIARRHEA" and filed with the China National Intellectual Property Administration on January 25, 2022**, and the prior application with the patent application No.** 202211339890.X entitled "USE OF BACTEROIDES FRAGILIS IN IMPROVEMENT AND TREATMENT OF DIARRHEA" and filed with the China National Intellectual Property Administration on October 28, 2022**, which are incorporated herein by reference in their entirety.**

### TECHNICAL FIELD

The present invention relates to the use of *Bacteroides fragilis,* specifically in the amelioration and/or treatment of diarrhea.

### BACKGROUND

Diarrhea is a gastrointestinal disease characterized by the copious and frequent (more than 3 times a day) passage of watery or soft stools compared to normal circumstances. In extreme cases, more than 20 liters of liquid may be lost in a single day. Moreover, it is a major health-threatening disease.. Although it might appear to be a minor ailment, its mortality rate ranks just behind conditions such as tumors and diabetes, placing it among the leading causes of death from various diseases. The data released by the World Health Organization in 2018 show that diarrheal disease is the second leading cause of death among children under 5 years of age worldwide, with about 525000 children under five years of age dying from it each year. The prevalence of diarrhea in adults should not be underestimated either. Most adults experience diarrhea at least once a year, and during outbreaks of intestinal infectious diseases, the impact is widespread, leading to even greater harm.

Diarrhea is not an independent disease but rather a common manifestation of many diseases. The etiologies of diarrhea can be classified into infectious diarrhea and non-infectious diarrhea. Infectious diarrhea is mostly caused by infections from viruses, bacteria, fungi, and parasites, and may be accompanied by symptoms such as abdominal pain, fever, and leukocytosis. The causes of non-infectious diarrhea include food poisoning, chemical poisoning, drug-induced diarrhea, dietary diarrhea, symptomatic diarrhea, allergic diarrhea, diarrhea associated with other congenital metabolic diseases, and the like.

Currently, the clinical treatment for diarrhea mainly focuses on symptomatic drug therapies, such as montmorillonite powder, loperamide, tannic acid protein, bismuth subcarbonate, aluminum hydroxide gel, and the like. Diarrhea can be classified into acute and chronic types, with symptoms lasting less than two weeks and at least two weeks, respectively. The first choice for treating infectious acute diarrhea is antibiotics (such as norfloxacin, levofloxacin, moxifloxacin, metronidazole, and the like) for etiological treatment. However, treatment using antibiotics alone can increase gastrointestinal side effects in patients and, in severe cases, may even worsen the disease condition. Therefore, it is necessary to supplement with antidiarrheal drugs for symptomatic treatment. The treatment of chronic diarrhea often involves long-term intermittent therapy, which includes dietary adjustments, supplemented with non-centrally acting drugs such as montmorillonite powder and bismuth subcarbonate, as well as microecological modulators such as live *Bifidobacterium* and live *Bacillus licheniformis.*

Symbiotic bacteria play an important role in maintaining intestinal homeostasis and offer protection to intestinal integrity. Therefore, the use of probiotics to treat diarrhea has become a research hotspot in recent years. Probiotics are a type of active microorganisms that are beneficial to a host, which alter the composition of flora at a certain part of the host by colonizing the human body. Paraprobiotics are defined as "non-viable microbial cells which, when administered orally or topically in sufficient amounts, confer benefits to consumers". This definition includes both morphologically incomplete microbial cells and morphologically intact microbial cells (see Tavemiti V, Guglielmetti S. The immunomodulatory properties of probiotic microorganisms beyond their viability (ghost probiotics: proposal of paraprobiotic concept) [J]. Genes & Nutrition, 2011, 6(3): 261-274.)). According to the mainstream research view, broken microbial cells can better release functional molecules and therefore have a better effect; however, for intact inactivated microbial cells, there is no unified view that can account for their superiority over live bacteria in certain applications. Currently, research on paraprobiotics is primarily focused on the first-generation probiotics such as *Lactobacillus* and *Bifidobacterium,* with relatively fixed research directions. Therefore, there is a need to develop novel paraprobiotics and broaden the indications for their use.

### SUMMARY

In view of the above problems, the present invention provides use of *Bacteroides fragilis* in the amelioration and/or treatment of diarrhea.

In order to better solve the above problems, the present invention adopts the following technical solutions:
In a first aspect, the present invention provides use of *Bacteroides fragilis* in the preparation of a composition for ameliorating and/or treating diarrhea, wherein the *Bacteroides fragilis* can be in the form of live bacteria or inactivated bacteria .

In an embodiment of the present invention, the *Bacteroides fragilis* is selected from *Bacteroides fragilis* ZY-312 with the preservation number CGMCC No. 10685.

According to an embodiment of the present invention, the diarrhea is infectious diarrhea or non-infectious diarrhea.

Preferably, the infectious diarrhea is one or more of viral infectious diarrhea, bacterial/fungal infectious diarrhea, and parasitic infectious diarrhea; and/or the non-infectious diarrhea is one or more of non-infectious inflammatory diarrhea, neoplastic diarrhea, malabsorption diarrhea, motility-associated diarrhea, intestinal dysbacteriosis-associated diarrhea, and drug-associated diarrhea. Preferably, the viral infectious diarrhea is one or more of norovirus, rotavirus, sapovirus, astrovirus, adenovirus, enterovirus, coronavirus, and the like; the bacterial infection is one or more of *Salmonella, Shigella, Campylobacter, Yersinia enterocolitica, Vibrio cholerae,* enterotoxigenic *Escherichia coli, Staphylococcus aureus,* diarrheagenic *Escherichia coli,* and the like; the fungal infection is *Candida, Mucor, Aspergillus,* and the like; the parasite infection includes *Giardia lamblia (Giardia), Entamoeba histolytica, Cryptosporidium cuniculus, Cyclospora, Blastocystis hominis, Trichinella,* and *Schistosoma* (complete complement); the malabsorption diarrhea is secondary small intestinal malabsorption diarrhea, wherein preferably, the secondary small intestinal malabsorption diarrhea is dyspeptic diarrhea, and further preferably, the dyspeptic diarrhea is lactose malabsorption diarrhea; and/or the intestinal dysbacteriosis-associated diarrhea is *Clostridium difficile* dysregulation-associated diarrhea; and/or the drug in the drug-associated diarrhea is one or more of a laxative (phenolphthalein, senna leaf, castor oil, or the like), a hyperosmotic drug (magnesium sulfate, sodium sulfate, or the like), an antibiotic (penicillins, cephalosporins, lincomycin, clindamycin, or the like), a chemotherapeutic drug (epirubicin, docetaxel, fluorouracil, hydroxycamptothecin, or the like), an antihypertensive drug (propranolol, reserpine, methyldopa, or the like), an antiarrhythmic drug (cardiac glycosides, quinidine, or the like), a diuretic drug (furosemide, ethacrynic acid, or the like), and a lipid-lowering drug (clofibrate, cholestyramine, or the like), and further preferably, the drug is one or more of a laxative, a hyperosmotic drug, and an antibiotic.

Preferably, the *Bacteroides fragilis* is the form of inactivated bacteria , and further preferably, the *Bacteroides fragilis* is the form of morphologically intact inactivated bacteria and/or morphologically incomplete inactivated bacteria .

Preferably, the *Bacteroides fragilis* described above is inactivated by any one or more of the methods including dry heat, moist heat, filtration, organic solvents, chemical reagents, ultraviolet or infrared radiation, fermentation, freeze drying, genetic recombination, and genetic modification or engineering.

Preferably, the *Bacteroides fragilis* is available as *Bacteroides fragilis* live bacteria powder or inactivated bacteria powder; the *Bacteroides fragilis* inactivated bacteria powder is prepared by the steps of fermentation culture, washing with an aqueous sodium chloride solution and centrifugation, addition of an excipient for resuspension, inactivation, and drying.

Further preferably, the *Bacteroides fragilis* inactivated bacteria powder is prepared by the following method, which comprises the following steps:
(1) fermentation culturing of *Bacteroides fragilis*;
(2) post-fermentation, centrifuging the fermentation liquid to collect bacterial cells, then washing these cells with an aqueous sodium chloride solution at a weight-to-volume ratio of 1 g:(10-30) mL, followed by centrifugation to obtain the washed bacterial cells;
(3) adding a first excipient solution to the washed bacterial cells to mix and resuspend, forming a bacterial cell solution, then performing inactivation treatment and centrifugation to collect an inactivated bacterial sludge;
(4) adding a second excipient solution to the inactivated bacterial sludge obtained in step (3) to obtain an inactivated bacteria stock solution; and
(5) drying the inactivated bacteria stock solution obtained in step (4) until a residual water content is less than 5 wt%, thus obtaining the *Bacteroides fragilis* inactivated bacteria powder.

According to an embodiment of the present invention, in step (2), a bacterial count in the fermentation liquid reaches no less than 10⁸ CFU/mL.

According to an embodiment of the present invention, in step (2), a mass concentration of the aqueous sodium chloride solution ranges from 0.6-1.5 wt%, preferably from 0.65-1.2 wt%, more preferably from 0.8-1.0 wt%, most preferably from 0.85-0.95 wt%, such as 0.9 wt%.

According to an embodiment of the present invention, the excipient includes at least one of mannitol, sorbitol, maltodextrin, lactose, sodium chloride, maltose, sucrose, glucose, trehalose, dextran, proline, lysine, alanine, casein, and skim milk.

According to an embodiment of the present invention, in step (3), a weight-to-volume ratio of the bacterial cells to the first excipient solution is 1 g:(5-40) mL.

According to an embodiment of the present invention, in step (3), the excipient in the first excipient solution has a mass fraction of 4-30 wt%, with the excipient defined as previously described. According to an embodiment of the present invention, in step (3), the solvent of the first excipient solution is selected from an aqueous sodium chloride solution, which are defined as previously described. Further preferably, the solvent of the first excipient solution is selected from normal saline, such as a 0.9 wt% aqueous sodium chloride solution.

According to an embodiment of the present invention, in step (3), the inactivation treatment is performed by at least one selected from heat inactivation, freeze inactivation, and chemical inactivation, preferably heat inactivation.

Illustratively, the heat inactivation is performed at a temperature of 60-100 °C for a period of 10-60 min.

According to an embodiment of the present invention, in step (4), the second excipient solution is added to allow the total weight of the inactivated bacteria stock solution to be consistent with the weight of the bacterial cell solution before the inactivation in step (3). Preferably, the second excipient solution is the same as or different from the first excipient solution.

According to an embodiment of the present invention, the excipient in the second excipient solution has a mass fraction of is 4-30 wt%,with the excipient defined as previously described.

Preferably, the solvent of the second excipient solution is selected from an aqueous sodium chloride solution, which are as defined previously. Further preferably, the solvent of the first excipient solution is selected from normal saline, such as a 0.9 wt% aqueous sodium chloride solution.

Illustratively, the second excipient solution is identical to the first excipient solution.

According to an embodiment of the present invention, in step (5), the drying is performed by a method selected from vacuum freeze drying and/or spray drying, preferably vacuum freeze drying. Illustratively, the vacuum freeze drying is performed under conditions comprising: a freezing temperature of -20 to -40 °C, a freezing time of 1-3 h, and a vacuum degree of 0.20-0.25 mbar. Illustratively, the vacuum freeze drying is performed by a process comprising: pre-freezing at - 40±2 °C for 1-3 h, then pre-freezing at -20±2 °C for 0.5-1 h, finally pre-freezing at -40±2 °C for another 0.5-2 h, and performing primary drying and desorption drying at a vacuum degree of 0.25 mbar to prepare the inactivated bacteria powder.

According to an embodiment of the present invention, in the preparation method, the centrifugation conditions are not specifically limited, provided that the desired centrifugation effect can be achieved; for example, the centrifugation is performed at a rotation speed of 10000-20000 rpm.

According to an embodiment of the present invention, in the application and/or use described above, the composition may be any one of a pharmaceutical composition, food, a healthcare product, or a food additive.

Preferably, in the application and/or use described above, the pharmaceutical composition comprises a pharmaceutically effective dose of the inactivated bacteria powder of the *Bacteroides fragilis* ZY-312 with the preservation number of CGMCC No. 10685.

In the pharmaceutical composition described above, the pharmaceutical composition is in the dosage form of a pill, a tablet, a granule, a capsule, a powder, a suspension, an oral liquid, an enema agent, or the like.

Preferably, the pharmaceutical composition is administered orally or by enema.

The administration cycle of the pharmaceutical composition described above is intermittent administration, periodic administration, continuous administration, or chronic administration.

Compared with the prior art, the present invention has the following beneficial effects.

Through different animal diarrhea model experiments, the present invention demonstrates that the live bacteria, lysate solution and inactivated bacteria of *Bacteroides fragilis,* particularly the *Bacteroides fragilis* ZY-312 with the preservation number of CGMCC No. 10685, have the effects of ameliorating and treating infectious diarrhea and non-infectious diarrhea. The inactivated bacteria powder of the morphologically intact *Bacteroides fragilis* provided by the present invention, in different concentration formulas, shows a better effect in ameliorating infectious diarrhea and non-infectious diarrhea and has no side effects on the body, thereby having good application prospects.

### BRIEF DESCRIPTION OF THE DRAWINGS

FIG. 1 is a picture showing the colony morphology of *Bacteroides fragilis* ZY-312 after anaerobic culture according to the present invention.
FIG. 2 is a gram-staining microscopic image of *Bacteroides fragilis* ZY-312 according to the present invention.
FIG. 3 is a transmission electron microscopy image of the *Bacteroides fragilis* ZY-312 inactivated bacteria powder according to the present invention.
FIGs. 4A-4E show the expression levels of interferon-stimulated genes (ISGs) in small intestinal tissues of various groups of suckling mice according to Example 10 of the present invention.
Note: *lfit1, lfit2, lfit3, Oasl2,* and *Rsad2* are interferon-stimulated genes (ISGs). * indicates *P* < 0.05, ** indicates *P* < 0.01, *** indicates *P* < 0.001, and **** indicates *P* < 0.0001, compared with the model group; ^{#} indicates *P* < 0.05, ^{##} indicates *P* < 0.01, and ^{###} indicates *P* < 0.001, compared with the NCTC 9343 live bacteria solution group; ⁺ indicates *P* < 0.05, ⁺ indicates *P* < 0.01, and ⁺⁺⁺ indicates P < 0.001, compared with the NCTC 9343 inactivated bacteria solution group (T tests).

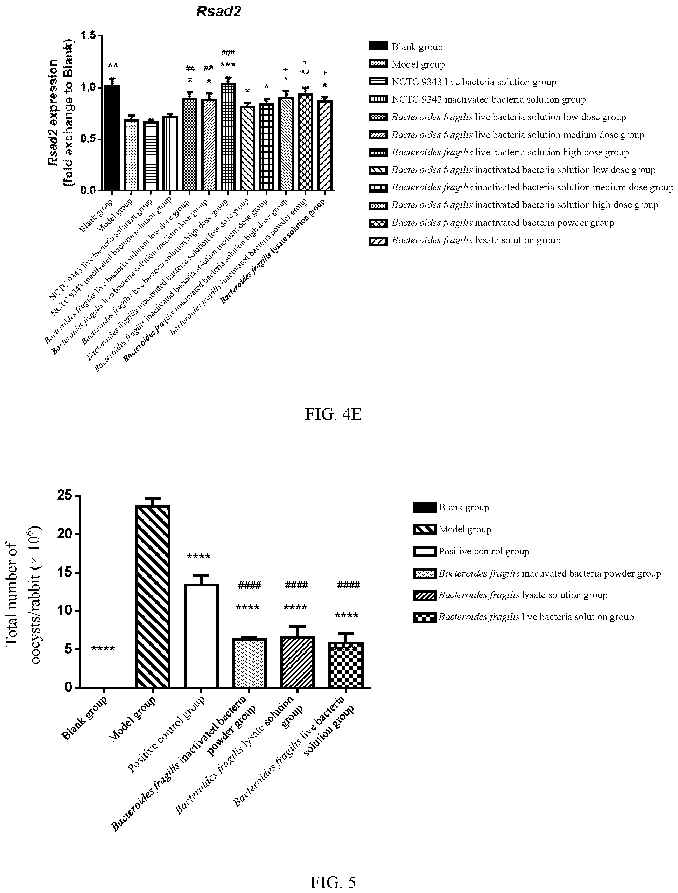

FIG. 5 is a bar chart showing the total number of oocysts per individual New Zealand rabbit in each group during the treatment period according to Example 12 of the present invention.

The microbial strains used in the implementation process of the present invention were preserved in the China General Microbiological Culture Collection Center (CGMCC), located at No. 3, Yard No. 1, West Beichen Road, Chaoyang District, Beijing, on April 2, 2015. The strain was Classified and named *Bacteroides fragilis* ZY-312, with the preservation number of CGMCC No. 10685. *Bacteroides fragilis* ZY-312 was independently isolated by the applicant of the present invention and is currenly protected under a granted patent (Patent No. 201510459408.X)..According to the Patent Examination Guidelines, there is no requirement for preservation, i.e., the provision of a preservation certificate is unnecessary, if the strain is commercially available to the public or has already been licensed.

### DETAILED DESCRIPTION

The technical solutions of the present invention will be further described in detail with reference to the following specific examples. It should be understood that the following examples are merely exemplary illustrations and explanations of the present invention and should not be construed as limiting the protection scope of the present invention. All techniques implemented based on the content of the present invention described above are encompassed within the protection scope of the present invention.

Unless otherwise stated, the starting materials and reagents used in the following examples are all commercially available products or can be prepared by using known methods.

In the following examples and comparative examples, unless otherwise specified, *Bacteroidesfragilis* is subjected to fermentation culture using methods known in the art, wherein the *Bacteroidesfragilis* is *Bacteroidesfragilis* ZY-312 with the preservation number of CGMCC No. 10685.

### Example 1. Fermentation Culture of Bacteroides Fragilis

The *Bacteroidesfragilis* ZY-312 strain was subjected to streak inoculation on a blood agar plate and then anaerobically cultured for 48 h. The morphological characteristics, staining characteristics, size, globular shape or rod-like shape, distribution, and the like of the colonies were observed.

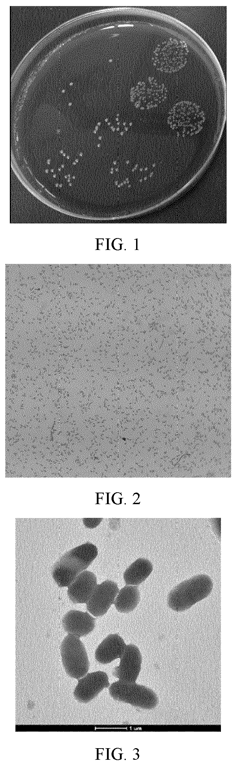

Colony characteristics:
After 48 hours of cultivation on blood agar,, the *Bacteroides fragilis* ZY-312 presented a round, slightly convex, translucent, and white, with a smooth surface and no hemolysis.. The diameter of the colonies was 1-3 mm, as shown in FIG. 1.

Morphology under a microscope:Upon Gram staining, *Bacteroides fragilis* ZY-312 was identified as a Gram-negative bacterium, displaying a typical rod shape with blunt and densely stained ends. The unstained part in the middle of the bacterial cell appeared as a bubble, as shown in FIG. 2.

A single colony was selected and inoculated in a culture medium for fermentation culture for 8 h (at a temperature of 37 °C), and the resulting bacteria solution was centrifuged for precipitation at a rotation speed of 3000 r/min for 15 min. The supernatant was then removed, and the precipitate was collected, thus obtaining a *Bacteroidesfragilis* ZY-312 bacterial sludge.

### Example 2. Preparation of Samples

I. Preparation of *Bacteroides fragilis* live bacteria solution, lysate and inactivated bacteria solution
   (1) Enrichment of bacteria: a single colony from the anaerobically cultured colonies in Example 1 was selected and inoculated in TSB (tryptone soy broth containing 5% fetal bovine serum) for enrichment fermentation culture, and the resulting bacteria solution was preserved for later use.
   (2) *Bacteroides fragilis* live bacteria solution: the bacteria solution prepared in step (1) was subjected to bacterial count determination by using a Mcfarland turbidimetric tube and was diluted to 10⁶ CFU/mL, 10⁷ CFU/mL, 10⁸ CFU/mL, 10⁹ CFU/mL, and 10¹⁰ CFU/mL with normal saline and preserved for later use.
   (3) *Bacteroides fragilis* lysate solution: the *Bacteroides fragilis* lysate solution was prepared by ultrasonication. Specific steps: 5 mL of the *Bacteroides fragilis* live bacteria solution prepared in step (2) was subjected to lysis for 30 min using an ultrasonic instrument (with a cycle of 10 seconds on and 10 seconds off), and this system achieved a bacterial cell lysis efficiency of 99%; after lysis, the lysed bacteria cells were centrifuged at 6000 rpm and 4 °C for 10 min and then filtered for later use.
   (4) *Bacteroides fragilis* inactivated bacteria solution: the *Bacteroides fragilis* inactivated bacteria solution was prepared by high temperature or ultraviolet irradiation. Specific steps: 5 mL of each of the *Bacteroides fragilis* live bacteria solutions at 10⁷ cells/mL and 10⁹ cells/mL prepared in step (2) was placed in a beaker, and the beaker containing the bacteria solution was left to stand in a constant-temperature water bath at 100 °C for 20-30 min or in an ultraviolet environment for 30-60 min, thus obtaining the *Bacteroides fragilis* inactivated bacteria solution.
II. Preparation of *Bacteroides fragilis* inactivated bacteria powders
   (1) The *Bacteroides fragilis* fermentation liquid prepared in Example 1 was taken and subjected to centrifugation, the wet bacterial cells were collected, and normal saline was added at a ratio of bacterial cells:normal saline = 1:(10-30) (m:v) to resuspend and wash the bacterial sludge; the washed bacterial cells were then recollected by centrifugation; m represents mass, and v represents volume.
   (2) An excipient prepared by mixing 5% maltodextrin with 0.9% sodium chloride was added to the bacterial cells obtained in step (1) at a ratio of bacterial cells:excipient = 1:(5-15) (m:m), and the mixture was stirred for dispersion, then subjected to heat inactivation at (70-100)±5 °C for (20-40)±5 min, followed by centrifugation to collect the bacterial sludge..
   (3) An excipient was added to the inactivated bacterial sludge collected in step (2) to allow the total weight to be consistent with the weight of the bacteria solution before inactivation, and the mixture was stirred to allow the bacterial sludge to be completely dissolved, thus obtaining an inactivated bacteria powder stock solution.
   (4) The inactivated bacteria powder stock solution obtained in step (3) underwent vacuum freeze-drying, involving a pre-freeze at -40±2°C for 1-3 hours, followed by a pre-freeze at -20±2°C for 0.5-1 hour, and another pre-freeze at -40±2°C for 0.5-2 hours. This was then subjected to primary drying at -5±2°C and 0±2°C, and desorption drying at 35±2°C under a vacuum of 0.25 mbar, to produce the inactivated bacteria powder. The bacterial count in the powder reached at least 1 × 10¹¹ cells/g. The microscopic image in Figure 3 shows that the bacteria cells in the produced inactivated bacteria powder were morphologically intact.

An NCTC 9343 inactivated bacteria powder was prepared using the same method as described above. The samples prepared in this example were for use in the following examples.

### Example 3. Effect of Bacteroides Fragilis Inactivated Bacteria Powder on Mice with Senna Leaf-Induced Diarrhea

### I. Experimental procedures

In this example, 60 SPF-grade Kunming mice weighing 18-22 g (half male and half female) were selected for the experiment. Each experimental mouse was assigned a unique identifier. Prior to grouping, the mouse cage labels were marked with the project number, species/strain, sex, cage number, and animal identifier. Using the BioBook software, the mice were randomly divided based on the initial weight and sex into the following 6 groups : a blank group (group 1), a model group (group 2), a positive control group (group 3, 0.0030 g/mL atropine sulfate solution), and low (group 4), medium (group 5) and high (group 6) dose groups receiving the *Bacteroides fragilis* ZY-312 inactivated bacteria powder obtained in Example 2, with each group comprising 10 mice. Preparation of senna leaf solution: senna leaves were weighed and boiled in water for about 10 min, the mixture was then filtered, and the filtrate was concentrated under reduced pressure to obtain a 1.33 g/mL medicinal solution.

Establishing senna leaf-induced diarrhea model: Mice were fasted for 4 h prior to administration of the senna leaf solution but had free access to water; 10 mice (blank group) were intragastrically given an equal amount of normal saline, and the remaining 50 mice were intragastrically given the 1.33 g/mL senna leaf medicinal solution at a dose of 15 µL/g body weight; the administration was carried out twice daily for 4 consecutive days. After the end of the modeling, the 50 molded mice were randomly divided into corresponding groups.

Administration regimen: except for the blank group (group 1) and the model group (group 2), which were intragastrically given an equal amount of normal saline, the other groups were administered the corresponding drug via a single intragastric gavage at a dosage of 10 µL/g body weight (approximately 0.2 mL); the treatment was carried out for 7 consecutive days. After the end of the treatment, the mice were placed in mouse cages with filter paper at the bottom, with 1 mouse per cage. The filter paper was replaced hourly over a 5-hour observation period. For each mouse, the number of loose stools and the total number of stools within every 1 h were recorded, and the loose stool rate, loose stool grade and diarrhea index were then calculated.

Mouse stools could be classified into 5 types: normal stools, stools with normal appearance but having high water content, soft stools with abnormal appearance, watery stools, and mucus stools. The first 2 types were considered normal stools, while the last 3 types were considered diarrheal stools. The distinction between dry and loose stools was made based on the presence or absence of marks on the filter paper. Stool frequency was counted as one occurrence per pellet or pile (when individual pellets could not be distinguished).

Diarrhea index = loose stool rate × loose stool grade, wherein the loose stool rate was the ratio of the number of loose stools to the total number of stools for each mouse. The loose stool grade was determined by the size of the stained area formed by loose stools contaminating filter paper and included 4 grades: less than 1 cm as grade 1, 1-1.9 cm as grade 2, 2-3 cm as grade 3, and greater than 3 cm as grade 4.

For compiling statistics, the grade of each pile of loose stools was individually counted, and the grades of all loose stools for that mouse were summed and then divided by the number of loose stools to obtain the mean grade of loose stools, namely the loose stool grade. Measurement of grade diameter: if the stool had a round shape, the diameter was measured directly; if the stool had an oval stool shape, the longest diameter and the diameter of the approximate circle were measured, summed, and then divided by 2.

The specific experimental groups and administration regimens are shown in Table 1.

**Table 1. Experimental groups and administration regimens for the effect of Bacteroides fragilis inactivated bacteria powder on mice with senna leaf-induced diarrhea**

| **Group** | **N** | **Test substance** | **Administration concentration** | **Administration volume (µL/g body weight)** | **Route of administration** | **Frequency of administration** |
|---|---|---|---|---|---|---|
| 1 | 10 | Normal saline + normal saline | N/A | 15+10 | i.g. | Bid*4 days+QD*7 days |
| 2 | 10 | Senna leaf + normal saline | 1.33 g/mL | 15+10 | i.g. | Bid*4 days+QD*7 days |
| 3 | 10 | Senna leaf + atropine sulfate | 1.33 g/mL+0.003 g/mL | 15+10 | i.g. | Bid*4 days+QD*7 days |
| 4 | 10 | Senna leaf + *Bacteroides fragilis* inactivated bacteria powder (low) | 1.33 g/mL+1×10⁶ Cells/mL | 15+10 | i.g. | Bid*4 days+QD*7 days |
| 5 | 10 | Senna leaf + *Bacteroides fragilis* inactivated bacteria powder (medium) | 1.33 g/mL+1×10⁸ Cells/mL | 15+10 | i.g. | Bid*4 days+QD*7 days |
| 6 | 10 | Senna leaf + *Bacteroides fragilis* inactivated bacteria powder (high) | 1.33 g/mL+1×10¹⁰ Cells/mL | 15+10 | i.g. | Bid*4 days+QD*7 days |

### II. Experimental results

**Table 2. Effect of Bacteroides fragilis inactivated bacteria powder on mice with senna leaf-induced diarrhea**

| **Group No.** | **Group** | **Test substance** | **Diarrhea index** |
|---|---|---|---|
| 1 | Blank group | N/A | 0.0±0.0** |
| 2 | Model group | Senna leaf | 2.71±0.12 |
| 3 | Positive control group | Senna leaf + atropine sulfate | 1.38±0.09** |
| 4 | Low dose group | Senna leaf + *Bacteroides fragilis* inactivated bacteria powder (low) | 1.95±0.16** |
| 5 | Medium dose group | Senna leaf + *Bacteroides fragilis* inactivated bacteria powder (medium) | 1.72±0.06** |
| 6 | High dose group | Senna leaf + *Bacteroides fragilis* inactivated bacteria powder (high) | 1.47±0.14** |

| | | | |
|---|---|---|---|
| Note: ** indicates *P* < 0.01 and * indicates *P* < 0.05, compared with the model group. | | | |

The results are shown in Table 2. The diarrhea indices of the positive control group and each dose group of the *Bacteroides fragilis* inactivated bacteria powder were all extremely significantly lower than that of the model group (*P* < 0.01). The results indicate that the *Bacteroides fragilis* inactivated bacteria powder provided by the present invention could effectively ameliorate symptoms of diarrhea in mice and exhibit a dose-response effect.

### Example 4. Effect of Bacteroides Fragilis on Mice with Senna Leaf-Induced Diarrhea

### I. Experimental procedures

The procedures followed in this experiment were similar to those in Example 3, with the following modifications: 70 SPF-grade Kunming mice were randomized into seven groups: a blank group (group 1), a model group (group 2), a positive control group (group 3, 4 mg/kg Imodium), and a live bacteria solution group (group 4), a lysate group (group 5), an inactivated bacteria solution group (group 6) and an inactivated bacteria powder group (group 7) of *Bacteroides fragilis,* with each group comprising 10 mice. Following 4 days of model induction through continuous intragastric administration of senna leaves" *Bacteroides fragilis* treatments was carried out twice daily (once in the morning and once in the afternoon) for 4 consecutive days. The mice were observed daily for behavioral characteristics, clinical symptoms, and the like, with a focus on monitoring and recording diarrhea condition. After the end of the treatment, the mice were dissected for pathological observation.

The specific experimental groups and administration regimens are shown in Table 3.

**Table 3. Experimental groups and administration regimens for the effect of Bacteroides fragilis on mice with senna leaf-induced diarrhea**

| **Group** | **N** | **Test substance** | **Administration concentration** | **Administration volume (µL/g body weight)** | **Route of administration** | **Frequency of administration** |
|---|---|---|---|---|---|---|
| 1 | 10 | Normal saline + normal saline | N/A | 15+10 | i.g. | Bid*4 days+Bid*4 days |
| 2 | 10 | Senna leaf + normal saline | 1.33 g/mL | 15+10 | i.g. | Bid*4 days+Bid*4 days |
| 3 | 10 | Senna leaf + Imodium | 1.33 g/mL+4 mg/kg | 15+10 | i.g. | Bid*4 days+Bid*4 days |
| 4 | 10 | Senna leaf + *Bacteroides fragilis* live bacteria solution | 1.33 g/mL+10⁹ CFU/mL | 15+10 | i.g. | Bid*4 days+Bid*4 days |
| 5 | 10 | Senna leaf + *Bacteroides fragilis* lysate solution | 1.33 g/mL+10⁹ Cells/mL | 15+10 | i.g. | Bid*4 days+Bid*4 days |
| 6 | 10 | Senna leaf + *Bacteroides fragilis* inactivated bacteria solution | 1.33 g/mL+10⁹ Cells/mL | 15+10 | i.g. | Bid*4 days+Bid*4 days |
| 7 | 10 | Senna leaf + *Bacteroides fragilis* inactivated bacteria powder | 1.33 g/mL+10¹⁰ Cells/mL | 15+10 | i.g. | Bid*4 days+Bid*4 days |

### II. Experimental results

**Table 4. Results of changes in the number of animals with soft stools/total number of animals in each group after Bacteroides fragilis treatment**

| **Group** | **Test substance** | **Treatment time (day)** | | | | |
|---|---|---|---|---|---|---|
| | | **D1** | **D2** | **D3** | **D4** | **D5** |
| Blank group | N/A | 0/10* | 0/10* | 0/10* | 0/10* | 0/10* |
| Model group | N/A | 10/10 | 10/10 | 10/10 | 10/10 | 10/10 |
| Positive control group | Imodium | 5/10 | 3/10* | 0/10* | 0/10* | 0/10* |
| Live bacteria solution group | *Bacteroides fragilis* live bacteria solution | 7/10 | 5/10 | 3/10* | 2/10* | 0/10* |
| Lysate solution group | *Bacteroides fragilis* lysate solution | 8/10 | 6/10 | 4/10* | 3/10* | 1/10* |
| Inactivated bacteria solution group | *Bacteroides fragilis* inactivated bacteria solution | 8/10 | 7/10 | 4/10* | 2/10* | 0/10* |
| Inactivated bacteria powder group | *Bacteroides fragilis* inactivated bacteria powder | 9/10 | 8/10 | 4/10* | 1/10* | 0/10* |

| | | | | | | |
|---|---|---|---|---|---|---|
| Note: * indicates P < 0.05, compared with the model group. | | | | | | |

As indicated in Table 4, the positive control group (treated with Imodium) demonstrated a significant therapeutic effect by the second day of treatment (*P* < 0.05) and achieved complete recovery by the third day; The groups treated with the *Bacteroides fragilis* live bacteria solution, lysate , inactivated bacteria solution and inactivated bacteria powder showed significant therapeutic effects by the third day (*P* < 0.05), and were essentially completely cured by the fifth day. The results indicate that the *Bacteroides fragilis* live bacteria solution, lysate, inactivated bacteria solution and inactivated bacteria powder provided by the present invention had an effect of significantly ameliorating symptoms of diarrhea in mice.

### Example 5. Effect of Bacteroides Fragilis on Mice with Magnesium Sulfate-Induced Diarrhea

### I. Experimental procedures

In this example, 70 SPF-grade Kunming mice weighing 18-22 g (half male and half female) were selected for the experiment. Each experimental mouse was assigned a unique identifier. Prior to grouping, the mouse cage labels were marked with the project number, species/strain, sex, cage number, and animal identifier. Using the BioBook software, the mice were randomly divided based on the initial weight and sex into the following 6 groups: a blank group (group 1), a model group (group 2), a positive control group (group 3, 0.8 g/mL magnesium sulfate solution), low (group 4), medium (group 5) and high (group 6) dose groups receiving the *Bacteroides fragilis* ZY-312 inactivated bacteria powder obtained in Example 2, and a group of the live bacteria obtained in Example 2 (group 7), with each group comprising 10 mice.

Modeling of magnesium sulfate-induced diarrhea model: Prior to magnesium sulfate administration, the mice were fasted for 4 h, with free access to water; 10 mice (blank group) were intragastrically given an equal amount of normal saline, and the remaining 60 mice were intragastrically given the 0.8 g/mL magnesium sulfate solution at a dose of 10 µL/g body weight; the administration was carried out twice daily for 4 consecutive days. After the end of the modeling, the 60 molded mice were randomly divided into corresponding groups.

Administration regimen: except for the blank group (group 1) and the model group (group 2), which were intragastrically given an equal amount of normal saline, the other groups were administered the corresponding drug via a single intragastric gavage at a dose of 10 µL/g body weight (about 0.2 mL); the treatment was carried out for 7 consecutive days. After the end of the treatment, the mice were placed in mouse cages with filter paper at the bottom, with 1 mouse per cage. The filter paper was replaced hourly over a 5-hour observation period. For each mouse, the number of loose stools and the total number of stools within every 1 h were recorded, and the loose stool rate, loose stool grade and diarrhea index were then calculated.

Mouse stools could be classified into 5 types: normal stools, stools with normal appearance but having high water content, soft stools with abnormal appearance, watery stools, and mucus stools. The first 2 types were considered normal stools, and the latter last 3 types were considered diarrheal stools. The distinction between dry and loose stools was made based on the presence or absence of marks on the filter paper. Stool frequency was counted as one occurrence per pellet or pile (when individual pellets could not be distinguished).

Diarrhea index = loose stool rate × loose stool grade, wherein the loose stool rate was the ratio of the number of loose stools to the total number of stools for each mouse. The loose stool grade was determined by the size of the stained area formed by loose stools contaminating filter paper and included 4 grades: less than 1 cm as grade 1, 1-1.9 cm as grade 2, 2-3 cm as grade 3, and greater than 3 cm as grade 4.

For compiling statistics, the grade of each pile of loose stools was individually counted, and the grades of all loose stools for that mouse were summed and then divided by the number of loose stools to obtain the mean grade of loose stools, namely the loose stool grade. Measurement of grade diameter: if the stool had a round shape, the diameter was measured directly; if the stool had an oval stool shape, the longest diameter and the diameter of the approximate circle were measured, summed, and then divided by 2. The specific experimental groups and administration regimens are shown in Table 5.

**Table 5. Experimental groups and administration regimens for the effect of Bacteroides fragilis on mice with magnesium sulfate-induced diarrhea**

| **Group** | **N** | **Test substance** | **Administration concentration** | **Administration volume (µL/g body weight)** | **Route of administration** | **Frequency of administration** |
|---|---|---|---|---|---|---|
| 1 | 10 | Normal saline + normal saline | N/A | 15+10 | i.g. | Bid*4 days+QD*7 days |
| 2 | 10 | MgSO₄ + normal saline | 0.8 g/mL | 15+10 | i.g. | Bid*4 days+QD*7 days |
| 3 | 10 | MgSO₄ + atropine sulfate | 0.8 g/mL+0.003 g/mL | 15+10 | i.g. | Bid*4 days+QD*7 days |
| 4 | 10 | MgSO₄ + *Bacteroides fragilis* inactivated bacteria powder (low) | 0.8 g/mL+1×10⁶ Cells/mL | 15+10 | i.g. | Bid*4 days+QD*7 days |
| 5 | 10 | MgSO₄ + *Bacteroides fragilis* inactivated bacteria powder (medium) | 0.8 g/mL+1×10⁸ Cells/mL | 15+10 | i.g. | Bid*4 days+QD*7 days |
| 6 | 10 | MgSO₄ + *Bacteroides fragilis* inactivated bacteria powder (high) | 0.8 g/mL+1×10¹⁰ Cells/mL | 15+10 | i.g. | Bid*4 days+QD*7 days |
| 7 | 10 | MgSO₄ + *Bacteroides fragilis* live bacteria solution | 0.8 g/mL+1×10¹⁰ CFU/mL | 15+10 | i.g. | Bid*4 days+QD*7 days |

### II. Experimental results

**Table 6. Effect of Bacteroides fragilis on mice with magnesium sulfate-induced diarrhea**

| **Group No.** | **Group** | **Test substance** | **Diarrhea index** |
|---|---|---|---|
| 1 | Blank group | N/A | 0.00±0.00** |
| 2 | Model group | MgSO₄ | 3.01±0.11 |
| 3 | Positive control group | MgSO₄ + atropine sulfate | 1.14±0.06** |
| 4 | Low-dose inactivated bacteria group | MgSO₄ + *Bacteroides fragilis* inactivated bacteria powder | 1.82±0.16** |
| 5 | Medium-dose inactivated bacteria group | MgSO₄ + *Bacteroides fragilis* inactivated bacteria powder | 1.51±0.14** |
| 6 | High-dose inactivated bacteria group | MgSO₄ + *Bacteroides fragilis* inactivated bacteria powder | 1.27±0.13**^{##} |
| 7 | Live bacteria group | MgSO₄ + *Bacteroides fragilis* live bacteria solution | 1.44±0.13** |

| | | | |
|---|---|---|---|
| Note: ** indicates *P* < 0.01 and * indicates *P* < 0.05, compared with the model group; ^{##} indicates *P* < 0.01, compared with the live bacteria group. | | | |

The experimental results show that the diarrhea indices of the positive control group, each dose group of the *Bacteroides fragilis* inactivated bacteria powder, and the live bacteria group were all extremely significantly lower than that of the model group (*P* < 0.01); however, the diarrhea index of the *Bacteroides fragilis* live bacteria group was significantly higher than that of the *Bacteroides fragilis* inactivated bacteria group at the same dose. The above results indicate that the *Bacteroides fragilis* inactivated bacteria powder provided by the present invention could effectively ameliorate symptoms of diarrhea in mice and achieved a dose-dependent response, and its drug effect was superior to that of the *Bacteroides fragilis* live bacteria.

### Example 6. Effect of Bacteroides Fragilis on Mice with Escherichia Coli-Induced Diarrhea

### I. Experimental procedures

(1) Preparation of bacteria solution
   *Escherichia coli* preserved on a slant was inoculated on a nutrient agar plate and cultured at 37 °C for 24 h. Several colonies were then picked, inoculated into MH broth, followed by another 24 hours of culture at 37 °C. The culture was then diluted with sterilized normal saline and subjected to turbidity adjustment using a 0.5 Mcfarland turbidimetric tube, thus achieving a bacterial content of 3 × 10⁸ CFU/mL.
(2) Therapeutic experiment on mice of *Escherichia coli* diarrhea model
   Mice weighing about 20 g were selected, and each mouse was intraperitoneally injected with 0.2 mL of the *Escherichia coli* bacteria solution with a bacterial content of 3 × 10⁸ CFU/mL for diarrhea induction. A positive control group received a 0.01 g/mL aqueous berberine hydrochloride solution administered intragastrically. The experimental grouping and procedures were the same as in Example 3. The mice were continuously observed for 5 h, and the diarrhea indices of the mice were recorded. The specific experimental groups and administration regimens are shown in Table 7.

**Table 7. Experimental groups and administration regimens for the effect of Bacteroides fragilis on mice with Escherichia coli-induced diarrhea**

| **Group** | **N** | **Test substance** | **Administration concentration** | **Administration volume (µL/g body weight)** | **Route of administration** | **Frequency of administration** |
|---|---|---|---|---|---|---|
| 1 | 10 | Normal saline + normal saline | N/A | 10+10 | i.p.+i.g. | Bid*4 days+QD*7 days |
| 2 | 10 | *Escherichia coli +* normal saline | 3×10⁸ CFU/mL | 10+10 | i.p.+i.g. | Bid*4 days+QD*7 days |
| 3 | 10 | *Escherichia coli +* berberine hydrochloride | 3×10⁸ CFU/mL+0.01 g/mL | 10+10 | i.p.+i.g. | Bid*4 days+QD*7 days |
| 4 | 10 | *Escherichia coli + Bacteroides fragilis* inactivated bacteria powder (low) | 3×10⁸ CFU/mL+1×10⁶ Cells/mL | 10+10 | i.p.+i.g. | Bid*4 days+QD*7 days |
| 5 | 10 | *Escherichia coli + Bacteroides fragilis* inactivated bacteria powder (middle) | 3×10⁸ CFU/mL+1×10⁸ Cells/mL | 10+10 | i.p.+i.g. | Bid*4 days+QD*7 days |
| 6 | 10 | *Escherichia coli + Bacteroides fragilis* inactivated bacteria powder (high) | 3×10⁸ CFU/mL+1×10¹⁰ Cells/mL | 10+10 | i.p.+i.g. | Bid*4 days+QD*7 days |
| 7 | 10 | *Escherichia coli + Bacteroides fragilis* live bacteria solution | 3×10⁸ CFU/mL+1×10¹⁰ CFU/mL | 10+10 | i.p.+i.g. | Bid*4 days+QD*7 days |

### II. Experimental results

**Table 8. Effect of Bacteroides fragilis on mice with Escherichia coli-induced diarrhea**

| **Group No.** | **Group** | **Test substance** | **Diarrhea index** |
|---|---|---|---|
| 1 | Blank group | N/A | 0.00±0.00** |
| 2 | Model group | *Escherichia coli* | 2.47±0.10 |
| 3 | Positive control group | *Escherichia coli* + berberine hydrochloride | 1.25±0.13** |
| 4 | Low-dose inactivated bacteria group | *Escherichia coli* + *Bacteroides fragilis* inactivated bacteria powder | 1.93±0.08** |
| 5 | Medium-dose inactivated bacteria group | *Escherichia coli* + *Bacteroides fragilis* inactivated bacteria powder | 1.57±0.11** |
| 6 | High-dose inactivated bacteria group | *Escherichia coli* + *Bacteroides fragilis* inactivated bacteria powder | 1.31±0.05**^{##} |
| 7 | Live bacteria group | *Escherichia coli* + *Bacteroides fragilis* live bacteria powder | 1.54±0.18** |

| | | | |
|---|---|---|---|
| Note: ** indicates *P* < 0.01 and * indicates *P* < 0.05, compared with the model group; ^{##} indicates *P* < 0.01, compared with the live bacteria group. | | | |

The results in Table 8 show that the diarrhea indices of the positive control group, each dose group of the *Bacteroides fragilis* inactivated bacteria powder, and the live bacteria group were all significantly lower than that of the model group (P < 0.01); however, the diarrhea index of the *Bacteroides fragilis* live bacteria group was significantly higher than that of the *Bacteroides fragilis* inactivated bacteria group at the same dose. The above results indicate that the *Bacteroides fragilis* inactivated bacteria powder provided by the present invention could effectively ameliorate symptoms of diarrhea and achieved a dose-dependent response.

### Example 7. Effect of Bacteroides Fragilis on Rats with Antibiotic-Induced Diarrhea

An antibiotic-associated diarrhea model was constructed using a combination of three antibiotics (clindamycin, ampicillin, and streptomycin) in a mass ratio of 1:1:1. Subsequently, a suspension of NCTC 9343 inactivated bacteria powder at 1 × 10⁹ cells/mL, a suspension of *Bacteroides fragilis* inactivated bacteria powder at 1 × 10⁹ cells/mL, and a suspension of *Bacteroides fragilis* live bacteria at 1 × 10⁹ CFU/mL (prepared in Example 2) were each used for treatment. The therapeutic effect of the *Bacteroides fragilis* inactivated bacteria powder was observed.

### I. Experimental procedures

Sixty female SD rats aged 7-8 weeks and weighing 200-250 g were selected and randomly divided into the following 6 groups: a blank group (group 1), a model group (group 2), a positive control group (group 3, 1.5 mg/kg Imodium), an NCTC 9343 inactivated bacteria powder group (group 4, 1 × 10⁹ cells/mL), a *Bacteroides fragilis* inactivated bacteria powder group (group 5, 1 × 10⁹ cells/mL), and a *Bacteroides fragilis* live bacteria group (group 6, 1 × 10⁹ CFU/mL), with each group comprising 10 rats.

Each animal in the groups was intraperitoneally injected with 0.2 mL of a 180 mg/mL triple antibiotic combination solution, while the blank group was injected with an equal amount of normal saline; the administration was carried out once daily for 7 consecutive days. From day 8, the corresponding groups of animals were intragastrically given (1 mL/100 g body weight) normal saline, a suspension of Imodium, a suspension of NCTC 9343 inactivated bacteria powder at 1 × 10⁹ cells/mL, a suspension of *Bacteroides fragilis* inactivated bacteria powder at 1 × 10⁹ cells/mL, and a suspension of *Bacteroides fragilis* live bacteria at 1 × 10⁹ CFU/mL, respectively; the administration was carried out twice daily (once in the morning and once in the afternoon) for 7 consecutive days. The rats were observed daily for behavioral characteristics, clinical symptoms, and the like. After treatment completion, the rats were dissected for pathological observation.

Fecal samples from each rat, housed in individual cages within were collected 4 h in the afternoon of day 7 (end of modeling) and the afternoon of day 14 (end of experiment).The stools were weighed for wet weight, and dried in an oven at 80 °C for 3 h before weighing again to determine the dry weight The moisture content of the stools was calculated using the formula: Water content of stools (%) = ((wet weight of stools - dry weight of stools) / wet weight of stools) × 100.

The specific experimental groups and administration regimens are shown in Table 9.

**Table 9. Experimental groups and administration regimens for the effect of Bacteroides fragilis on rats with antibiotic-induced diarrhea**

| **Group** | **N** | **Test substance** | **Administration concentration** | **Administration volume** | **Route of administration** | **Frequency of administration** |
|---|---|---|---|---|---|---|
| 1 | 10 | Normal saline + normal saline | N/A | 0.2 mL + 1 mL/100 g body weight | i.p.+i.g. | qd*7 days +bid*7 days |
| 2 | 10 | Triple antibiotic combination + normal saline | 180 mg/mL | 0.2 mL + 1 mL/100 g body weight | i.p.+i.g. | qd*7 days +bid*7 days |
| 3 | 10 | Triple antibiotic combination + Imodium | 180 mg/mL+1.5 mg/kg | 0.2 mL + 1 mL/100 g body weight | i.p.+i.g. | qd*7 days +bid*7 days |
| 4 | 10 | Triple antibiotic combination + NCTC 9343 inactivated bacteria powder | 180 mg/mL+1×10⁹ Cells/mL | 0.2 mL + 1 mL/100 g body weight | i.p.+i.g. | qd*7 days +bid*7 days |
| 5 | 10 | Triple antibiotic combination + *Bacteroides fragilis* inactivated | 180 mg/mL+1×10⁹Cells/mL | 0.2 mL + 1 mL/100 g body weight | i.p.+i.g. | qd*7 days +bid*7 days |
| | | bacteria powder | | | | |
| 6 | 10 | Triple antibiotic combination + *Bacteroides fragilis* live bacteria solution | 180 mg/mL+1×10⁹ CFU/mL | 0.2 mL + 1 mL/100 g body weight | i.p.+i.g. | qd*7 days +bid*7 days |

### II. Experimental results

**Table 10. Effect of Bacteroides fragilis on rats with antibiotic-induced diarrhea**

| **Group No.** | **Group** | **Water content of stools (%)** | |
|---|---|---|---|
| | | **End of modeling** | **End of experiment** |
| 1 | Blank group | 53.6±3.0** | 47.85±5.36*** |
| 2 | Model group | 73.7±6.2 | 71.33±4.62 |
| 3 | Positive control group (Imodium) | 72.6±2.1 | 55.26±6.33*** |
| 4 | NCTC 9343 inactivated bacteria powder group | 69.9±3.1 | 65.53±4.47* |
| 5 | *Bacteroides fragilis* inactivated bacteria powder group | 71.8±3.2 | 57.92±5.81*** ^{##@} |
| 6 | *Bacteroides fragilis* live bacteria group | 71.67±2.29 | 62.72±3.90*** |

| | | | |
|---|---|---|---|
| Note:*** indicates *P* < 0.001, ** indicates *P* < 0.01, and * indicates *P* < 0.05, compared with the model group; ^{##} indicates *P* < 0.01, compared with the NCTC9343 inactivated bacteria powder group; ^{@} indicates *P* < 0.05, compared with the *Bacteroides fragilis* live bacteria powder group. | | | |

The results in Table 10 show that the water content of stools of the *Bacteroides fragilis* inactivated bacteria powder group was comparable to that of the positive control group, and both were significantly lower than that of the model group (*P* < 0.001); the water content of stools of the NCTC 9343 inactivated bacteria powder group was relatively high, but was still significantly lower than that of the model group (*P* < 0.05); the water content of stools of the *Bacteroides fragilis* live bacteria powder group was significantly lower than that of the model group (*P* < 0.001), but had statistical significance as compared with the *Bacteroides fragilis* inactivated bacteria powder group (*P* < 0.05). The above results indicate that the *Bacteroides fragilis* inactivated bacteria powder provided by the present invention could effectively ameliorate symptoms of antibiotic-associated diarrhea, and its therapeutic effect intensity was comparable to that of Imodium and superior to that of the NCTC 9343 inactivated bacteria powder group (P < 0.01) and the *Bacteroides fragilis* live bacteria group (P < 0.05).

### Example 8. Effect of Bacteroides Fragilis on Clostridium Difficile-Induced Diarrhea in Mice

A *Clostridium difficile-induced* diarrhea model was constructed using intraperitoneal injection of clindamycin in combination with intragastric administration of *Clostridium difficile.* Subsequently, treatments were applied using a 1 × 10⁹ cells/mL suspension of NCTC 9343 inactivated bacteria powder, a 1 × 10⁹ cells/mL suspension of *Bacteroides fragilis* inactivated bacteria powder, and a 1 × 10⁹ CFU/mL suspension of *Bacteroides fragilis* live bacteria powder (prepared in Example 2). The therapeutic effect of the *Bacteroides fragilis* inactivated bacteria powder was observed.

### I. Experimental procedures

### 1. Preparation of bacteria solution

*Clostridium difficile* preserved on a slant was inoculated on a CCFA agar plate and cultured at 37 °C for 72 h in an incubator with an anaerobic gas mixture (80% N₂-10% CO₂-10% H₂). Several colonies were then picked, inoculated into CCFA broth, and cultured at 37 °C for 72 h. The culture was then diluted with sterilized normal saline and subjected to turbidity adjustment using a 0.5 Mcfarland turbidimetric tube, thus achieving a bacterial content of 3 × 10⁸ CFU/mL.

### 2. Therapeutic experiment on mice of Clostridium difficile diarrhea model

Sixty female C57BL/6 mice aged 6-8 weeks and weighing 18-22 g were selected and randomly divided into the following 6 groups: a blank group (group 1), a model group (group 2), a positive control group (group 3, 4 mg/kg Imodium), an NCTC 9343 inactivated bacteria powder group (group 4, 1 × 10⁹ cells/mL), a *Bacteroides fragilis* inactivated bacteria powder group (group 5, 1 × 10⁹ cells/mL), and a *Bacteroides fragilis* live bacteria group (group 6, 1 × 10⁹ CFU/mL), with each group containing 10 mice.

Each group of animals was intraperitoneally injected with a clindamycin solution at 100 mg/kg, while the blank group was injected with an equal amount (0.1 mL/10 g body weight) of normal saline; the administration was carried out once daily for 3 consecutive days. From day 4, a *Clostridium difficile* bacteria solution at 3 × 10⁸ CFU/mL was intragastrically administered once daily for 7 consecutive days. From day 11, the corresponding groups of animals were intragastrically given (0.1 mL/10 g body weight) normal saline, a suspension of Imodium, a suspension of NCTC 9343 inactivated bacteria powder at 1 × 10⁹ ells/mL, and a suspension of *Bacteroides fragilis* inactivated bacteria powder at 1 × 10⁹ cells/mL, respectively; the administration was carried out twice daily (once in the morning and once in the afternoon) for 7 consecutive days.

The mice were observed daily for behavioral characteristics, clinical symptoms, and the like. Each mouse was observed daily for diarrhea condition, and reference was made to the diarrhea scoring methods in the study of Kurita A et al. for the diarrhea scoring criteria. 0 points: normal stools or no stools; 1 point: mild diarrhea, slightly wet and soft stools; 2 points: moderate diarrhea, wetter and shapeless stools, with mild perianal contamination; 3 points: severe diarrhea, watery stools, with severe perianal contamination (see Kurita A, Kado S, Kaneda N, et al. Modified irinotecan hydrochloride (CPT-11) administration schedule improves induetion of delayed-onset diarrhea in rats [J]. Cancer Chem other Pharmacol, 2000, 46(3): 211-220)).

After the treatment was completed, in the morning of day 18, the mice were euthanized and then dissected for pathological observation. The pathological scoring criteria are as follows (see Talamisu TSUKAHARA, Yoshie IWASAKI, Keizo NAKAYAMA et al. Microscopic structure of the large intestinal mucosa in piglets during an antibiotic-associated diarrhea [J]. vet. Med. Sci, 2003, 65(3): 301-306.):
(1) Edema
   0 points: no edema.
   1 point: mild edema with only minor (< 2 X) multiple submucosal expansion.
   2 points: moderate edema with moderate (2-3 X) multiple submucosal expansion.
   3 points: severe edema with massive (> 3 X) multiple submucosal expansion.
   4 points: severe edema with diffuse submucosal expansion.
(2) Inflammatory cell infiltration
   0 points: no inflammation.
   1 point: minor multifocal neutrophil infiltration.
   2 points: moderate multifocal neutrophil infiltration (involving more of the submucosa layer).
   3 points: massive multifocal or even aggregated neutrophil infiltration (involving more of the submucosa and muscularis layers).
   4 points: lesion involvement similar to that for 3 points, but with abscess formation or more extensive involvement of the muscularis layer.
(3) Intestinal epithelial injury
   0 points: no changes in intestinal epithelial injury.
   1 point: minor multifocal superficial epithelial injury (vacuolation, apoptosis of some cells, attenuation/necrosis of villus tip).
   2 points: moderate multifocal superficial epithelial injury (vacuolation, apoptosis of some cells, attenuation/necrosis of villus tip).
   3 points: massive multifocal epithelial injury vacuolation, apoptosis of some cells, attenuation/necrosis of villus tip) ± pseudomembrane formation (fibrous exudate containing neutrophils and sloughed epithelium in the lumen).
   4 points: more pronounced pseudomembrane formation or epithelial ulceration (completely sloughed epithelium at the lesion site) in addition to the criteria for 3 points.

### II. Experimental results

**Table 11. Effect of Bacteroides fragilis on diarrhea scores in mice induced by Clostridium difficile**

| **Group No.** | **Group** | **Diarrhea score** | |
|---|---|---|---|
| | | **End of modeling** | **End of experiment** |
| 1 | Blank group | 0.0±0.0** | 0.0±0.0** |
| 2 | Model group | 2.3±0.7 | 2.7±0.5 |
| 3 | Positive control group (Imodium) | 2.2±1.2 | 1.4±0.5** |
| 4 | NCTC 9343 inactivated bacteria powder group | 2.4±0.7 | 1.8±1.1* |
| 5 | *Bacteroides fragilis* inactivated bacteria powder group | 2.3±0.5 | 1.6±0.5** |
| 6 | *Bacteroides fragilis* live bacteria group | 2.3±0.7 | 1.7±0.5** |

| | | | |
|---|---|---|---|
| Note: ** indicates P < 0.01 and * indicates P < 0.05, compared with the model group. | | | |

**Table 12. Effect of Bacteroides fragilis on pathological score changes in mice with Clostridium difficile-induced diarrhea**

| **Group No.** | **Group** | **Pathological score** |
|---|---|---|
| 1 | Blank group | 0.0±0.0** |
| 2 | Model group | 7.2±0.92 |
| 3 | Positive control group (Imodium) | 2.9±0.99** |
| 4 | NCTC 9343 inactivated bacteria powder group | 5.2±1.51* |
| 5 | *Bacteroides fragilis* inactivated bacteria powder group | 3.6±0.94** ^{##@} |
| 6 | *Bacteroides fragilis* live bacteria group | 4.4±0.58** |

| | | |
|---|---|---|
| Note: ** indicates *P* < 0.01, and * indicates *P* < 0.05, compared with the model group; ^{##} indicates P < 0.01, compared with the NCTC 9343 inactivated bacteria powder group; ^{@} indicates *P* < 0.05, compared with the *Bacteroides fragilis* live bacteria group. | | |

The results in Tables 11 and 12 show that the diarrhea scores and pathological scores of the positive control group and the *Bacteroides fragilis* inactivated bacteria powder group were significantly lower than those of the model group (*P* < 0.01); the diarrhea scores and pathological score of the NCTC 9343 inactivated bacteria powder group were relatively high, but were also significantly lower than those of the model group (*P* < 0.05); the diarrhea scores and pathological score of the *Bacteroides fragilis* live bacteria powder group were significantly lower than those of the model group (*P* < 0.01), but the pathological score had statistical significance as compared with the *Bacteroides fragilis* inactivated bacteria powder group (*P* < 0.05). The above results indicate that the *Bacteroides fragilis* inactivated bacteria powder provided by the present invention could effectively ameliorate symptoms of *Clostridium difficile* infectious diarrhea, and its therapeutic effect was relatively strong and was superior to that of the NCTC 9343 inactivated bacteria powder (P < 0.01) and the *Bacteroides fragilis* live bacteria powder (P < 0.05).

### Example 9. Effect of Bacteroides Fragilis on High Lactose-Induced Chronic Diarrhea in Mice

A chronic diarrhea model was constructed by feeding Wistar rats with high-lactose feed for 21 days. Subsequently, treatment were adminstrated using the *Bacteroides fragilis* live bacteria solution, lysate, inactivated bacteria solution and inactivated bacteria powder prepared in Example 2. The therapeutic effect of *Bacteroides fragilis* on chronic diarrhea was observed.

### I. Experimental procedures

### 1. Preparation of feed

The feed was formulated according to the AOAC recommended formula (see: The Official Methods of Analysis of the AOAC. 14th ed. US, 1984, 877), as detailed in Table 13.

**Table 13. Feed ingredient content (%)**

| | Casein | Lactose | Starch | Sucrose | Corn oil | Cellulose | Vitamin^{a} | Inorganic salt^{b} | DL-methionine |
|---|---|---|---|---|---|---|---|---|---|
| Basic feed | 18 | 0 | 58.8 | 10 | 5 | 3 | 1 | 4 | 0.2 |
| High-lactose feed | 18 | 58.8 | 0 | 10 | 5 | 3 | 1 | 4 | 0.2 |

| | | | | | | | | | |
|---|---|---|---|---|---|---|---|---|---|
| Note: a indicates that 1 kg of the basic feed contained: 20000 IU of vitamin A (dry, stable), 2000 IU of vitamin D (dry, stable), 100 IU of vitamin E (dry, stable), 5 mg of vitamin K, 2000 mg of choline, 100 mg of inositol, 100 mg of *p*-aminobenzoic acid, 40 mg of niacin. 40 mg of calcium pantothenate, 5 mg of vitamin B₁, 8 mg of vitamin B₂, 5 mg of vitamin B₆, 0.03 mg of vitamin B₁₂, 2 mg of folic acid, and 0.4 mg of biotin. b represents that 1 kg of the basic feed contained: 194.5 g of KH₂PO₄, 190.7 g of CaCO₃, 69.6 g of NaCl, 28.65 g of MgSO₄, 13.5 g of FeSO₄•7H₂O, 2.05 g of MnSO₄•H₂O, 0.395 g of KI, 0.274 g of ZnSO₄•7H₂O, 0.239 g of CuSO₄•5H₂O, and 0.012 g of CoCl₂•6H₂O. | | | | | | | | | |

### 2. Therapeutic experiment on mice with high lactose-induced chronic diarrhea

Eighty SPF-grade Wistar young rats aged 3-4 weeks (half male and half female) were selected, subjected to adaptive feeding for 1 week, and then housed in separate cages with filter paper and an iron frame at the bottom. The rats were then randomly divided into the following 2 groups: a blank group (group 1, 10 rats) and a modeling group (70 rats). The young rats in the control group were fed with the basic feed, and the modeling group was fed with the high-lactose feed. After 21 days of modeling, a chronic diarrhea model was constructed, and 60 young rats with chronic diarrhea were randomly divided into a model group (group 2), a spontaneous recovery group (group 3), a positive control group (group 4, 300 mg/kg Zhengchangsheng), and a live bacteria solution group (group 5), a lysate group (group 6), an inactivated bacteria solution group (group 7) and an inactivated bacteria powder group (group 8) of *Bacteroides fragilis,* with each group comprising 10 mice. From day 22, the rats were subjected to intragastric administration (0.1 mL/10 g body weight) according to their groups, twice daily (once in the morning and once in the afternoon) for 14 consecutive days. The mice were observed daily for behavioral characteristics, clinical symptoms, and the like, with a focus on monitoring and recording diarrhea condition. On day 35, after the rats were anesthetized, blood was collected from the abdominal aorta, and the rats were dissected for pathological observation.

The specific experimental groups and administration regimens are shown in Table 14.

**Table 14. Experimental groups and administration regimens for the effect of Bacteroides fragilis on rats with high lactose-induced chronic diarrhea**

| **No.** | **Group** | **Administration regimen** |
|---|---|---|
| 1 | Blank group | Fed with basic feed, intragastrically given normal saline. |
| 2 | Model group | Fed with high-lactose feed, intragastrically given normal saline. |
| 3 | Spontaneous recovery group | Fed with basic feed, intragastrically given normal saline. |
| 4 | Positive control group (Zhengchangsheng) | Fed with high-lactose feed, intragastrically given Zhengchangsheng at 300 mg/kg. |
| 5 | *Bacteroides fragilis* live bacteria solution group | Fed with high-lactose feed, intragastrically given *Bacteroides fragilis* live bacteria solution at 1 × 10⁸ CFU/mL |
| 6 | *Bacteroides fragilis* lysate solution group | Fed with high-lactose feed, intragastrically given *Bacteroides fragilis* lysate solution at 1 × 10⁸ cells/mL |
| 7 | *Bacteroides fragilis* inactivated bacteria solution group | Fed with high-lactose feed, intragastrically given *Bacteroides fragilis* inactivated bacteria solution at 1 × 10⁸ cells/mL |
| 8 | *Bacteroides fragilis* inactivated bacteria powder group | Fed with high-lactose feed, intragastrically given suspension of *Bacteroides fragilis* inactivated bacteria powder at 1 × 10⁸ cells/mL |

As in Example 3, the mice were observed daily for behavioral characteristics, clinical symptoms, and the like, and the diarrhea condition and scores of each Wistar rat were recorded. At the conclusion of the conclusion of the experiment, the serum IL-6 levels were measured.

### II. Experimental results

**Table 15. Effect of Bacteroidesfragilis on body weights and diarrhea scores in Wistar rats with high lactose-induced chronic diarrhea**

| **Group No.** | **Group** | **Body weight (g)** | | **Diarrhea score** | |
|---|---|---|---|---|---|
| | | **End of modeling** | **End of experiment** | **End of modeling** | **End of experiment** |
| 1 | Blank group | 211±10*** | 283±16*** | 0.0±0.0** | 0.0±0.0** |
| 2 | Model group | 131±7 | 149±9 | 2.7±0.7 | 3.4±0.6 |
| 3 | Spontaneous recovery group | 130±8 | 157±8 | 2.6±0.7 | 3.1±0.5 |
| 4 | Positive control group (Zhengchangsheng) | 132±6 | 248±10*** | 2.7±0.7 | 1.9±1.0** |
| 5 | *Bacteroides fragilis* live bacteria solution group | 131±7 | 241±9*** | 2.6±0.9 | 1.7±0.9** |
| 6 | *Bacteroides fragilis* lysate solution group | 131±7 | 244±13*** | 2.6±0.7 | 1.8±0.3** |
| 7 | *Bacteroides fragilis* inactivated bacteria solution group | 130±4 | 237±10*** | 2.6±0.7 | 1.9±0.7** |
| 8 | *Bacteroides fragilis* inactivated bacteria powder group | 131±7 | 234±10*** | 2.6±0.7 | 2.1±0.6** |

| | | | | | |
|---|---|---|---|---|---|
| Note: * indicates *P* < 0.05, ** indicates *P* < 0.01, and *** indicates *P* < 0.001, compared to the model group. | | | | | |

**Table 16. Effect of Bacteroides fragilis on serum IL-6 in Wistar rats with high lactose-induced chronic diarrhea**

| **Group No.** | **Group** | **IL-6 (pg/mL)** |
|---|---|---|
| 1 | Blank group | 88.10±14.32** |
| 2 | Model group | 240.36±30.71 |
| 3 | Spontaneous recovery group | 219.04±14.10 |
| 4 | Positive control group (Zhengchangsheng) | 134.81±9.93** |
| 5 | *Bacteroides fragilis* live bacteria solution group | 135.24±12.09** |
| 6 | *Bacteroides fragilis* lysate solution group | 140.16±10.53** |
| 7 | *Bacteroides fragilis* inactivated bacteria solution group | 138.56±12.09** |
| 8 | *Bacteroides fragilis* inactivated bacteria powder group | 141.07±11.72** |

| | | |
|---|---|---|
| Note: * indicates P < 0.05 and ** indicates P < 0.01, compared with the model group. | | |

The results in Tables 15 and 16 show that after the end of the experiment, the body weights of rats in the positive control group and all *Bacteroides fragilis* groups were significantly higher than those in the model group, while the diarrhea scores and the serum IL-6 levels of these groups were significantly lower than those of the model group (P < 0.01), indicating that the *Bacteroides fragilis* provided by the present invention could effectively ameliorate symptoms of chronic diarrhea, and the mechanism may be related to the reduction of the expression of proinflammatory factor IL-6; it was also found that there were no significant differences in body weights, diarrhea scores, and IL-6 levels between the *Bacteroides fragilis* groups and the positive control group, indicating that the therapeutic effect of the *Bacteroides fragilis* provided by the present invention in treating chronic diarrhea was comparable to that of Zhengchangsheng (*Bacillus licheniformis* live bacteria capsules).

### Example 10. Effect of Bacteroides Fragilis on Rotavirus-Induced Diarrhea in mice

Rotavirus is the main cause of severe dehydrating gastroenteritis in children aged 5 years, with a high incidence in autumn and winter. The binding sites for host cell receptors are VP4 protein and VP7 protein. A viral diarrhea model was constructed using intragastric administration of the rotavirus Wa strain in mice is adopted to construct a viral diarrhea model. Subsequently, the NCTC 9343 live bacteria solution/inactivated bacteria solution, the *Bacteroides fragilis* ZY-312 live bacteria solution/inactivated bacteria solution, and the *Bacteroides fragilis* ZY-312 inactivated bacteria powder and lysate solution were each used for treatment. The therapeutic effects of the *Bacteroides fragilis* live bacteria solution, inactivated bacteria solution, inactivated bacteria powder and lysate solution in treating rotavirus-induced diarrhea in mice were evaluated.

### I. Experimental procedures

SPF-grade Kunming suckling mice aged 3 days (half male and half female) were selected and randomly divided into the following 10 groups: a blank group (group 1), a model group (group 2), an NCTC 9343 live bacteria solution group (group 3, 1 × 10⁹ CFU/mL), an NCTC 9343 inactivated bacteria solution group (group 4, 1 × 10⁹ cells/mL), a *Bacteroides fragilis* live bacteria solution low dose group (group 5, 1 × 10⁵ CFU/mL), a *Bacteroides fragilis* live bacteria solution medium dose group (group 6, 1 × 10⁷ CFU/mL), a *Bacteroides fragilis* live bacteria solution high dose group (group 7, 1 × 10⁹ CFU/mL), a *Bacteroides fragilis* inactivated bacteria solution low dose group (group 8, 1 × 10⁵ cells/mL), a *Bacteroides fragilis* inactivated bacteria solution medium dose group (group 9, 1 × 10⁷ cells/mL), a *Bacteroides fragilis* inactivated bacteria solution high dose group (group 10, 1 × 10⁹ cells/mL), a *Bacteroides fragilis* inactivated bacteria powder group (group 11, 1 × 10⁹ cells/mL), and a *Bacteroides fragilis* lysate solution group (group 12, 1 × 10⁹ cells/mL), with 8 mice in each group. After 1 hour of fasting treatment under constant temperature, each suckling mouse in groups 2 to 12 was orally inoculated with 50 µL of Wa strain rotavirus solution filtered through a 0.22 µm membrane with a titer of 1 × 10⁷ PFU, and each suckling mouse in group 1 was intragastrically given 50 µL of PBS to serve as a control. Twenty-four hours after the challenge by intragastric administration, suckling mice in the treatment groups exhibited yellow watery stools. The stools were collected to prepare a stool suspension, and then 100 µL of supernatant was pipetted using a pipette and added dropwise and vertically into a sample loading well of a rotavirus colloidal gold detection kit (Abon Biopharm (Hangzhou) Co., Ltd.). Within 10-20 min, red bands appeared in both the quality control and detection areas, indicating a positive result for rotavirus and successful modeling. Therefore, treatment could be carried out twice daily for 7 consecutive days. Daily observations were made for behavioral characteristics and clinical symptoms, particularly noting any cases of diarrhea. Stool samples were collected and centrifuged at the end of the modeling and treatment periods. The supernatants were taken for ELISA (Millipore) detection to determine the amount of rotavirus antigen in the stools. At the endpoint of the experiment, the mice were sacrificed using the cervical dislocation method, and small intestinal tissues were taken for RNA extraction. The expression of interferon-stimulated genes (ISGs) was then detected using the qPCR method. The specific experimental groups and administration regimens are shown in Table 17.

**Table 17. Experimental groups and administration regimens**

| **Group** | **N** | **Test substance** | **Administration concentration** | **Administration volume (µL)** | **Route of administration** | **Frequency of administration** |
|---|---|---|---|---|---|---|
| 1 | 8 | PBS+PBS | N/A | 50+50 | i.g. | QD*1 day + Bid*7 days |
| 2 | 8 | Rotavirus Wa strain + PBS | 1×10⁷ PFU | 50+50 | i.g. | QD*1 day + Bid*7 days |
| 3 | 8 | Rotavirus Wa strain + NCTC 9343 live bacteria solution | 1×10⁷ PFU +1×10⁹ CFU /mL | 50+50 | i.g. | QD*1 day + Bid*7 days |
| 4 | 8 | Rotavirus Wa strain + NCTC 9343 inactivated bacteria solution | 1×10⁷ PFU +1×10⁹ Cells/mL | 50+50 | i.g. | QD*1 day + Bid*7 days |
| 5 | 8 | Rotavirus Wa strain + *Bacteroides fragilis* live bacteria solution (low) | 1×10⁷ PFU +1×10⁵ CFU/mL | 50+50 | i.g. | QD*1 day + Bid*7 days |
| 6 | 8 | Rotavirus Wa strain + *Bacteroides fragilis* live bacteria solution (medium) | 1×10⁷ PFU +1×10⁷ CFU /mL | 50+50 | i.g. | QD*1 day + Bid*7 days |
| 7 | 8 | Rotavirus Wa strain + *Bacteroides fragilis* live bacteria solution (high) | 1×10⁷ PFU +1×10⁹ CFU /mL | 50+50 | i.g. | QD*1 day + Bid*7 days |
| 8 | 8 | Rotavirus Wa strain + *Bacteroides fragilis* inactivated bacteria | 1×10⁷ PFU +1×10⁵ Cells/mL | 50+50 | i.g. | QD*1 day + Bid*7 days |
| | | solution (Low) | | | | |
| 9 | 8 | Rotavirus Wa strain + *Bacteroides fragilis* inactivated bacteria solution (medium) | 1×10⁷ PFU +1×10⁷ Cells/mL | 50+50 | i.g. | QD*1 day + Bid*7 days |
| 10 | 8 | Rotavirus Wa strain + *Bacteroides fragilis* inactivated bacteria solution (high) | 1×10⁷ PFU +1×10⁹ Cells/mL | 50+50 | i.g. | QD*1 day + Bid*7 days |
| 11 | 8 | Rotavirus Wa strain + *Bacteroides fragilis* inactivated bacteria powder | 1×10⁷ PFU +1×10⁹ Cells/mL | 50+50 | i.g. | QD*1 day + Bid*7 days |
| 12 | 8 | Rotavirus Wa strain + *Bacteroides fragilis* lysate solution | 1×10⁷ PFU +1×10⁹ Cells/mL | 50+50 | i.g. | QD*1 day + Bid*7 days |

### II. Experimental results

The diarrhea scores of the suckling mice at the start of the treatment and after 7 days of treatment are shown in Table 18, and the amount of rotavirus antigen in the stools is shown in Table 19.

### Example 18. Effect of Bacteroides fragilis on diarrhea scores in mice induced by rotavirus

| **Group No.** | **Group** | **Diarrhea score (mean ± SD)** | |
|---|---|---|---|
| | | **End of modeling** | **End of experiment** |
| 1 | Blank group | 0.0±0.0** | 0.0±0.0** |
| 2 | Model group | 2.7±0.7 | 3.4±0.6 |
| 3 | NCTC 9343 live bacteria solution group | 2.6±0.7 | 2.1±0.5*** |
| 4 | NCTC 9343 inactivated bacteria solution group | 2.7±0.7 | 1.9±0.6*** |
| 5 | *Bacteroides fragilis* live bacteria solution low dose group | 2.6±0.9 | 1.3±0.2**** ^{###} |
| 6 | *Bacteroides fragilis* live bacteria solution medium dose group | 2.6±0.7 | 1.4±0.5**** ^{#} |
| 7 | *Bacteroides fragilis* live bacteria solution high dose group | 2.6±0.7 | 0.7±0.4**** ^{####} |
| 8 | *Bacteroides fragilis* inactivated bacteria solution low dose group | 2.6±0.7 | 1.2±0.6**** ⁺ |
| 9 | *Bacteroides fragilis* inactivated bacteria solution medium dose group | 2.6±0.8 | 1.3±0.3**** ⁺ |
| 10 | *Bacteroides fragilis* inactivated bacteria solution high dose group | 2.6±0.5 | 0.6±0.2**** ⁺⁺⁺⁺ |
| 11 | *Bacteroides fragilis* inactivated bacteria powder group | 2.7±0.5 | 0.8±0.4**** ⁺⁺⁺ |
| 12 | *Bacteroides fragilis* lysate solution group | 2.6±0.6 | 0 9±0 3**** ⁺⁺⁺ |

| | | | |
|---|---|---|---|
| Note: *** indicates *P* < 0.001 and **** indicates *P* < 0.0001, compared with the model group; ^{#} indicates *P* < 0.05, ^{###} indicates *P* < 0.001, and ^{####} indicates *P* < 0.0001, compared with the NCTC 9343 live bacteria solution group; ⁺ indicates *P* < 0.05 and ⁺⁺⁺ indicates *P* < 0.001, compared with the NCTC 9343 inactivated bacteria solution group (T tests). | | | |

**Table 19. Amount of antigen in stools**

| **Group No.** | **Group** | **Amount of antigen in stools (EU/mL, mean ± SD)** | |
|---|---|---|---|
| | | **End of modeling** | **End of experiment** |
| 1 | Blank group | 0.0±0.0*** | 0.0±0.0*** |
| 2 | Model group | 135.0±41.7 | 135.0±41.6 |
| 3 | NCTC 9343 live bacteria solution group | 135.0±41.7 | 110.0±37.5 |
| 4 | NCTC 9343 inactivated bacteria solution group | 135.0±41.7 | 120.0±31.7 |
| 5 | *Bacteroides fragilis* live bacteria solution low dose group | 135.0±41.7 | 55.0±27.8*** ^{##} |
| 6 | *Bacteroides fragilis* live bacteria solution medium dose group | 135.0±41.7 | 60.0±31.0*** ^{#} |
| 7 | *Bacteroides fragilis* live bacteria solution high dose group | 135.0±41.7 | 45.0±30.8*** ^{##} |
| 8 | *Bacteroides fragilis* inactivated bacteria solution low dose group | 135.0±41.7 | 50.0±28.7*** ⁺⁺⁺ |
| 9 | *Bacteroides fragilis* inactivated bacteria solution medium dose group | 135.0±41.7 | 55.0±30.3*** ⁺⁺⁺ |
| 10 | *Bacteroides fragilis* inactivated bacteria solution high dose group | 135.0±41.7 | 40.0±27.9*** ⁺⁺⁺ |
| 11 | *Bacteroides fragilis* inactivated bacteria powder group | 135.0±41.7 | 41.2±28.9*** ⁺⁺⁺ |
| 12 | *Bacteroides fragilis* lysate solution group | 135.0±41.7 | 47.3±32.7*** ⁺⁺⁺ |

| | | | |
|---|---|---|---|
| Note: *** indicates *P* < 0.001, compared with the model group; ^{#} indicates P < 0.05 and ^{##} indicates *P* < 0.01, compared with the NCTC 9343 live bacteria solution group; ⁺⁺⁺ indicates *P* < 0.001, compared with the NCTC 9343 inactivated bacteria solution group (T tests). | | | |

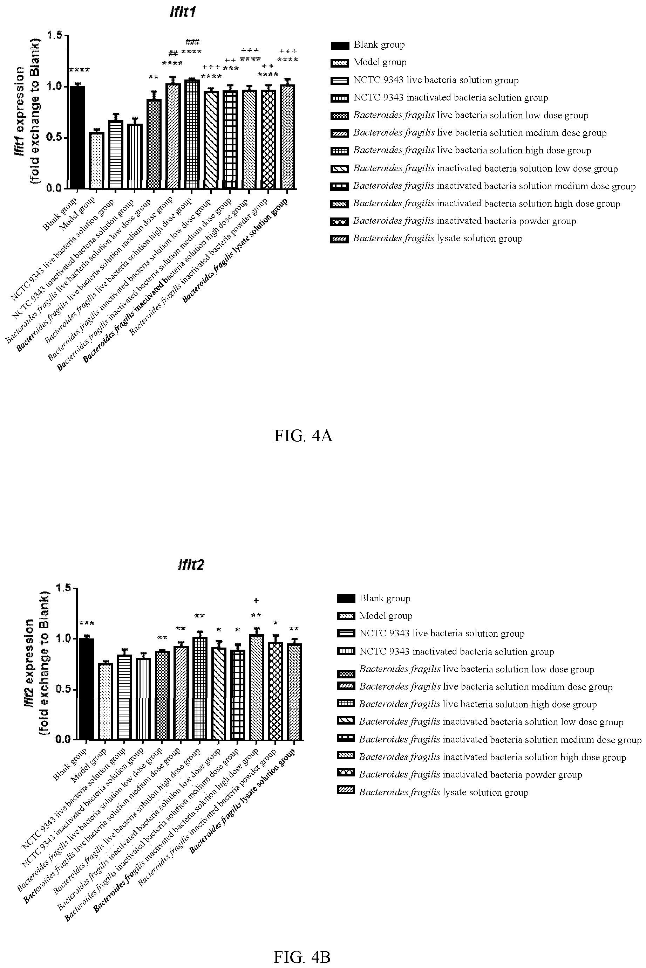

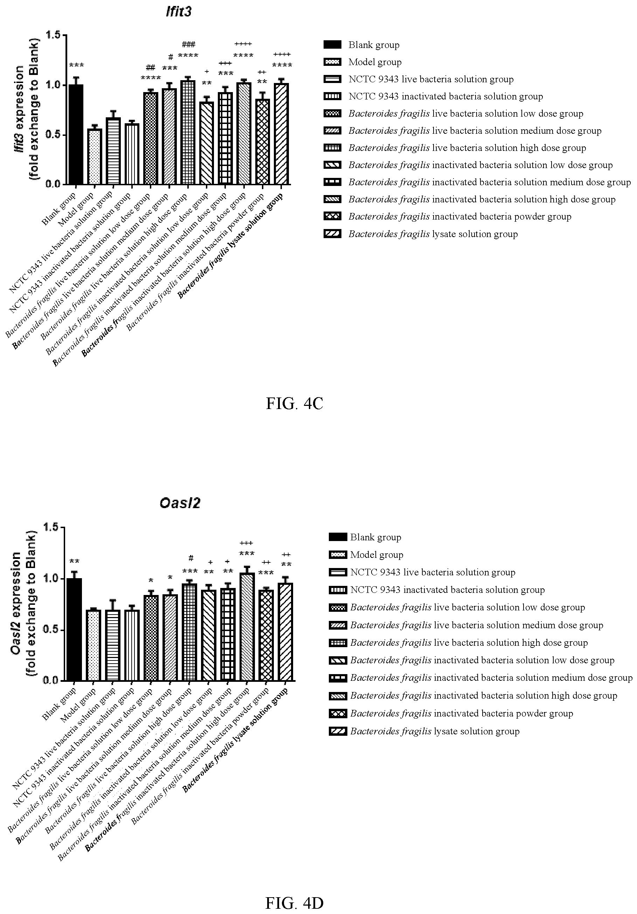

According to the data in Tables 18 and 19, it can be seen that after the end of the experiment, the diarrhea scores of rats in the NCTC 9343 live bacteria and inactivated bacteria groups (P < 0.05) and all *Bacteroides fragilis* ZY-312 groups (P < 0.0001) were significantly lower than that in the model group, indicating that the *Bacteroides fragilis* ZY-312 provided by the present invention could effectively ameliorate symptoms of virus-induced diarrhea; it was also found that the amount of rotavirus antigen in the stools of the *Bacteroides fragilis* live bacteria solution, inactivated bacteria solution, inactivated bacteria powder and lysate solution groups was significantly reduced compared with the model group (P < 0.001), while there were no significant differences between the NCTC 9343 live bacteria and inactivated bacteria groups and the model group, indicating that the amelioration of diarrhea by the *Bacteroides fragilis* ZY-312 may be related to the reduction of virus antigen load in the body, and the severity of diarrhea can be reduced by reducing the virus antigen load in the host. Meanwhile, the results in FIG. 4 show that compared with the model group, the expression levels of interferon-stimulated genes (ISGs) in all *Bacteroides fragilis* ZY-312 groups were significantly increased (P < 0.05); the expression of ISGs drove an antiviral state in both infected and neighboring uninfected cells, directly interfering with viral replication and spread. These results indicate that *Bacteroides fragilis* could up-regulate the expression of ISGs, initiating dendritic cell-mediated connections between innate and adaptive immunity through interferon-related pathways; this process induced a restrictive antiviral state in cells, induced apoptosis of infected cells, regulated immune cell subpopulations essential for antiviral responses, and coordinated the host's immune responses to the virus, thereby generating strong antiviral activity and thus ameliorating diarrhea. The effects in all *Bacteroides fragilis* ZY-312 groups were superior to those in the NCTC 9343 live bacteria and inactivated bacteria groups (P < 0.05). The amelioration effects of the low, medium and high doses of the *Bacteroides fragilis* ZY-312 bacteria solutions on viral diarrhea were as follows: high dose > medium dose ≈ low dose, with the effect of the live bacteria being comparable to that of the inactivated bacteria.

### Example 11. Resistance Effect of Bacteroides Fragilis on Norovirus Infection

Human norovirus causes more than 90% of non-bacterial gastroenteritis globally, with high incidences in spring and autumn. It leads to severe morbidity and mortality worldwide. The two main genotypes that infect humans are GI and GII. After a human body is selected to take encapsulated viral particles, the virus is spread through the fecal-oral route. After an incubation period of 24-48 hours, the virus can cause symptomatic diarrhea and vomiting within the next 12-60 hours. The norovirus mainly infects individuals aged 5-17 years and adults. In addition to causing diarrhea, it can also lead to upper respiratory tract symptoms. The binding site of the norovirus is the P structure of the VP1 protein, which is responsible for recognizing the host's histoblood group antigen (HBGA) receptors. Blocking binding of the norovirus to the HBGAs can reduce the virus's attachment to host cells, thereby weakening its infectivity to the host and thus ameliorating the diarrhea it causes.

### I. Experimental procedures

*Escherichia coli* BL21 competent cells were used to express the GII.17 type norovirus (NoV) P protein (GenBank accession No. KU557839), and the protein size and concentration were determined by SDS-PAGE electrophoresis. A 96-well ELISA plate was coated with boiled human A-type/B-type saliva diluted at 1:1000 in PBS (100 µL/well), incubated overnight, and then blocked with 5% skim milk. P protein (GII.17) (1 µg/mL) was pre-incubated with *Bacteroides fragilis* serially diluted with 1% skim milk at 37 °C for 1 h, and then the mixture was added to the 96-well plate; 100 µL of mouse anti-NoV polyclonal serum (1:3000, self-made) was then added into each well, and the mixture was incubated at 37 °C for 1 h. An HRP-goat anti-mouse IgG antibody (1:6000, Abcam) was then added, and the mixture was incubated at 37% for 1 h. All the above steps involved washing 5 times with 0.05% PBS-Tween 20. A substrate buffer containing TMB (freshly prepared, 100 µL/well) was added, and the mixture was left to stand in the dark at room temperature for 10 min for chromogenic reaction. The reaction was then terminated by adding 50 µL of 2 mol/L H₂SO₄. The OD values were measured at a wavelength of 450 nm on a microplate reader. Experimental groups and administration regimens are shown in Table 20, and the administration concentrations of the *Bacteroides fragilis* ZY-312 bacteria solutions are shown in Table 21.

**Table 20. Experimental groups for Bacteroides fragilis against norovirus**

| **Group** | **Saliva coating stage (µL/well) (12 h)** | **Pre-incubation stage (1 h)** | **Experimental system (culture volume: 100 µL/well, 4 replicate wells/group)** | | |
|---|---|---|---|---|---|
| | | | **Transfer to 96-well plate (µL/well)** | **Mouse anti-NoV polyclonal serum (µL/well) (1 h)** | **HRP-goat anti-mouse IgG antibody (µL/well) (1 h)** |
| Positive control group | 100 | / | P protein | 100 | 100 |
| *Bacteroides fragilis* live bacteria solution 1 experimental group | 100 | P protein + *Bacteroides fragilis* live bacteria solution 1 | At the end of the pre-incubation, P protein and *Bacteroides fragilis* were added together to the 96-well plate | 100 | 100 |
| *Bacteroides fragilis* live bacteria solution 2 experimental group | 100 | P protein + *Bacteroides fragilis* live bacteria solution 2 | | 100 | 100 |
| *Bacteroides fragilis* live bacteria solution | 100 | P protein + *Bacteroides fragilis* live | | 100 | 100 |
| 3 experimental group | | bacteria solution 3 | | | |
| *Bacteroides fragilis* live bacteria solution 4 experimental group | 100 | P protein + *Bacteroides fragilis* live bacteria solution 4 | | 100 | 100 |
| *Bacteroides fragilis* live bacteria solution 5 experimental group | 100 | P protein + *Bacteroides fragilis* live bacteria solution 5 | | 100 | 100 |
| *Bacteroides fragilis* inactivated bacteria solution M1 experimental group | 100 | P protein + *Bacteroides fragilis* inactivated bacteria solution M1 | | 100 | 100 |
| *Bacteroides fragilis* inactivated bacteria solution M2 experimental group | 100 | P protein + *Bacteroides fragilis* inactivated bacteria solution M2 | | 100 | 100 |
| *Bacteroides fragilis* inactivated bacteria solution M3 experimental group | 100 | P protein + *Bacteroides fragilis* inactivated bacteria solution M3 | | 100 | 100 |
| *Bacteroides fragilis* inactivated bacteria solution M4 experimental group | 100 | P protein + *Bacteroides fragilis* inactivated bacteria solution M4 | | 100 | 100 |
| *Bacteroides fragilis* inactivated bacteria solution M5 experimental group | 100 | P protein + *Bacteroides fragilis* inactivated bacteria solution M5 | | 100 | 100 |

**Table 21. Administration concentrations of Bacteroides fragilis live and inactivated bacteria solutions**

| **No.** | | **Administration concentration** |
|---|---|---|
| ***Bacteroides fragilis* live bacteria solution** | ***Bacteroides fragilis* inactivated bacteria solution** | |
| *Bacteroides fragilis* live bacteria solution 1 | *Bacteroides fragilis* inactivated bacteria solution M1 | 1×10⁵ Cells/mL |
| *Bacteroides fragilis* live bacteria solution 2 | *Bacteroides fragilis* inactivated bacteria solution M2 | 1×10⁶ Cells/mL |
| *Bacteroides fragilis* live bacteria solution 3 | *Bacteroides fragilis* inactivated bacteria solution M3 | 1×10⁷ Cells/mL |
| *Bacteroides fragilis* live bacteria solution 4 | *Bacteroides fragilis* inactivated bacteria solution M4 | 1×10⁸ Cells/mL |
| *Bacteroides fragilis* live bacteria solution 5 | *Bacteroides fragilis* inactivated bacteria solution M5 | 1×10⁹ Cells/mL |

### II. Experimental results

The ability of *Bacteroides fragilis* to block binding of NoV-HBGAs was evaluated by the blocking rate calculated as follows: blocking rate = (1 - mean OD₄₅₀ of experimental group/mean OD₄₅₀ of positive control group) × 100%. The results are shown in Table 22.

**Table 22. Blocking rate of Bacteroides fragilis against binding of NoV-HBGAs**

| **Group** | **Concentration of *Bacteroides fragilis*** | **Mean OD₄₅₀** | **Blocking rate (mean% ± SEM)** |
|---|---|---|---|
| Positive control group | / | 1.019 | / |
| *Bacteroides fragilis* live bacteria solution 1 experimental group | 1×10⁵ CFU/mL | 0.899 | 11.85±0.018** |
| *Bacteroides fragilis* live bacteria solution 2 experimental group | 1×10⁶ CFU/mL | 0.824 | 19.21±0.032*** |
| *Bacteroides fragilis* live bacteria solution 3 experimental group | 1×10⁷ CFU/mL | 0.660 | 35.30±0.032**** |
| *Bacteroides fragilis* live bacteria solution 4 experimental group | 1×10⁸ CFU/mL | 0.602 | 40.99±0.032**** |
| *Bacteroides fragilis* live bacteria solution 5 experimental group | 1×10⁹ CFU/mL | 0.550 | 46.04±0.034**** |
| *Bacteroides fragilis* inactivated bacteria solution M1 experimental group | 1×10⁵ Cells/mL | 0.929 | 8.9±0.023* |
| *Bacteroides fragilis* inactivated bacteria solution M2 experimental group | 1×10⁶ Cells/mL | 0.819 | 19.7±0.013**** |
| *Bacteroides fragilis* inactivated bacteria solution M3 experimental group | 1×10⁷ Cells/mL | 0.686 | 32.67±0.005**** |
| *Bacteroides fragilis* inactivated bacteria solution M4 experimental group | 1×10⁸ Cells/mL | 0.625 | 38.70±0.013**** |
| *Bacteroides fragilis* inactivated bacteria solution M5 experimental group | 1×10⁹ Cells/mL | 0.549 | 46.11 ± 0.014**** |

| | | | |
|---|---|---|---|
| Note: * indicates P < 0.05, ** indicates P < 0.01, *** indicates P < 0.001, and **** indicates P < 0.0001, compared with the positive control group (T tests). | | | |

The results in Table 22 show that compared with the positive control group, all *Bacteroides fragilis* groups were able to block binding of NoV-HBGAs (P < 0.05), with a significant reduction in OD₄₅₀ values (P < 0.05); the blocking rate of *Bacteroides fragilis* at 1 × 10⁹ cells/mL was approximately 50% (P < 0.0001). Moreover, as the administration concentration of *Bacteroides fragilis* increased, the OD₄₅₀ value decreased, and the ability to block binding of NoV-HBGAs became stronger, indicating that the *Bacteroides fragilis* could dose-dependently reduce the infectivity of the virus, demonstrating strong antiviral activity against the norovirus. Therefore, the *Bacteroides fragilis* has amelioration and resistance effects on diseases caused by the norovirus, such as diarrhea. The amelioration effects of the low, medium and high doses of the *Bacteroides fragilis* bacteria solutions on viral diarrhea were as follows: high dose > medium dose > low dose, with the effect of the live bacteria being comparable to that of the inactivated bacteria.

### Example 12. Effect of Bacteroides Fragilis on Diarrhea in Immunoactive Rabbits Infected with Cryptosporidium cuniculus

A parasite-associated diarrhea model was constructed by intragastric administration of *Cryptosporidium cuniculus* oocysts (extracted from a single positive stool sample from a rabbit farm in Jilin) in New Zealand white rabbits. Subsequently, the *Bacteroides fragilis* live bacteria solution, inactivated bacteria powder and lysate solution (prepared in Example 2) were each used for treatment. The therapeutic effects of the *Bacteroides fragilis* live bacteria solution, inactivated bacteria powder and lysate solution were observed.

### I. Experimental procedures

New Zealand white rabbits aged 35 days were selected and randomly divided into the following 6 groups: a blank group (group 1), a model group (group 2), a positive control group (nitazoxanide) (group 3), a *Bacteroides fragilis* inactivated bacteria powder group (group 4), a *Bacteroides fragilis* lysate solution group (group 5), and a *Bacteroides fragilis* live bacteria solution group (group 6), with 8 rabbits in each group. The New Zealand white rabbits in groups 2 to 6 were intragastrically inoculated with 1.5 × 10⁴ *Cryptosporidium cuniculus* oocysts, and the blank group was intragastrically given normal saline. Three days after the intragastric administration, the New Zealand rabbits exhibited yellow watery stools. The stools were collected to prepare a stool suspension, and structures with oocysts were observed under a microscope, indicating successful modeling. Therefore, treatment could be carried out. The blank group was given normal saline, and the treatment groups were subjected to drug administration. The treatment was carried out twice daily for 7 consecutive days. The New Zealand white rabbits were observed daily for diarrhea condition. On day 1 of the treatment and at the end of the treatment, the stool samples were collected, and the number of oocysts was observed under a microscope. The specific experimental groups and administration regimens are shown in Table 23.

**Table 23. Experimental groups and administration regimens for Bacteroides fragilis live bacteria, lysate solution and inactivated bacteria powder treating Cryptosporidium cuniculus-induced diarrhea in New Zealand rabbits**

| **Group** | **N** | **Test substance** | **Administration concentration** | **Administration volume (µL)** | **Route of administration** | **Frequency of administration** |
|---|---|---|---|---|---|---|
| 1 | 8 | Normal saline + normal saline | N/A | 100+100 | i.g. | QD*1 day + Bid*7 days |
| 2 | 8 | *Cryptosporidium cuniculus* oocysts + normal saline | 1.5 × 10⁴ oocysts | 100+100 | i.g. | QD*1 day + Bid*7 days |
| 3 | 8 | *Cryptosporidium cuniculus* oocysts + nitazoxanide | 1.5 × 10⁴ oocysts + 10 mg/kg | 100+100 | i.g. | QD*1 day + Bid*7 days |
| 4 | 8 | *Cryptosporidium cuniculus* oocysts + *Bacteroides fragilis* inactivated bacteria powder | 1.5 × 10⁴ oocysts + 1 × 10⁸ cells/mL | 100+100 | i.g. | QD*1 day + Bid*7 days |
| 5 | 8 | *Cryptosporidium cuniculus* oocysts + *Bacteroides fragilis* lysate solution | 1.5 × 10⁴ oocysts + 1 × 10⁸ cells/mL | 100+100 | i.g. | QD*1 day + Bid*7 days |
| 6 | 8 | *Cryptosporidium cuniculus* oocysts + *Bacteroides fragilis* live bacteria solution | 1.5 × 10⁴ oocysts + 1 × 10⁸ cells/mL | 100+100 | i.g. | QD*1 day + Bid*7 days |

### II. Experimental results

The number of oocysts in the daily stools of New Zealand rabbits during the administration period is shown in Table 24, and the total number of oocysts per New Zealand rabbit during the treatment period is shown in FIG. 5.

**Table 24. Number of oocysts in the daily stools of New Zealand rabbits during the administration period**

| **Group** | **Number of oocysts in stools (× 10⁶ oocysts/rabbit, mean ± SD)** | | | | | | |
|---|---|---|---|---|---|---|---|
| | **D1** | **D2** | **D3** | **D4** | **D5** | **D6** | **D7** |
| Blank group | 0±0 | 0±0 | 0±0 | 0±0 | 0±0 | 0±0 | 0±0 |
| Model group | 2.0±0.5 | 1.9±0.3 | 5.1±0.4 | 6.7±0.9 | 3.6±0.6 | 2.5±0.5 | 1.8±0.3 |
| Positive control group (nitazoxanide) | 1.8±0.6 | 3.2±1.5 | 2.8±0.9 | 2.2±0.2 | 1.5±1.1** | 1.3±0.1* | 0.6±0.2** |
| *Bacteroides fragilis* inactivated bacteria powder group | 1.7±1.4 | 1.0±1.1 | 1.5±0.6 | 0.5±1.0 | 0.3±1.3 | 0.6±0.2* | 0.2±0.1** |
| *Bacteroides fragilis* lysate solution group | 1.9±1.0 | 1.2±0.4* | 1.4±1.2* | 0.8±0.2** | 0.4±0.2** | 0.5±0.2** | 0.3±0.1** |
| *Bacteroides fragilis* live bacteria solution group | 1.9±0*** | 1.7±0*** | 1.2±0*** | 0.7±0.2** | 0.3±0.3** | 0.5±0.1** | 0±0**** |

| | | | | | | | |
|---|---|---|---|---|---|---|---|
| Note: * indicates P < 0.05, ** indicates P < 0.01, *** indicates P < 0.001, and **** indicates P < 0.0001, compared with the model group (T tests). | | | | | | | |

According to the data in Table 24, it can be seen that after the end of the experiment, the number of oocysts in the stools of New Zealand rabbits in the positive control group (nitazoxanide) and all *Bacteroides fragilis* groups was significantly lower than that in the model group (P < 0.01), indicating that the *Bacteroides fragilis* provided by the present invention could effectively ameliorate symptoms of *Cryptosporidium cuniculus*-induced diarrhea; in the *Bacteroides fragilis* live bacteria solution group, the number of oocysts in the stools of New Zealand rabbits was zero at the endpoint, indicating that the *Bacteroides fragilis* live bacteria solution could significantly ameliorate parasite-associated diarrhea (P < 0.0001). According to FIG. 5, it can be seen that during the treatment period, the total number of oocysts in the stools of each new Zealand rabbit in the positive control group (nitazoxanide) and all *Bacteroides fragilis* groups was significantly lower than that in the model group (P < 0.0001), and the total number of oocysts in the stools of each new Zealand rabbit in the *Bacteroides fragilis* lysate solution, live bacteria solution and inactivated bacteria powder groups was significantly lower than that in the positive control group (nitazoxanide) (P < 0.0001), indicating that the *Bacteroides fragilis* had a superior therapeutic effect on *Cryptosporidium cuniculus*-induced diarrhea compared to the existing drug nitazoxanide.

The above examples only illustrate several embodiments of the present invention for the purpose of specific and detailed description, but should not be construed as limiting the scope of the present invention. It should be noted that various changes and modifications can be made by those of ordinary skills in the art without departing from the spirit of the present invention, and these changes and modifications are all within the scope of the present invention. Therefore, the protection scope of the present invention should be determined by the appended claims.

## Claims

1. Use of *Bacteroides fragilis* in the preparation of a composition for ameliorating and/or treating diarrhea, wherein the *Bacteroides fragilis* is in the form of live bacteria, inactivated bacteria or a lysate.

2. The use according to claim 1, wherein the *Bacteroides fragilis* is selected from *Bacteroides fragilis* ZY-312 with the deposit number CGMCC No. 10685.

3. The use according to claim 1 or 2, wherein the diarrhea is infectious diarrhea or non-infectious diarrhea;
preferably, the infectious diarrhea comprises one or more of viral infectious diarrhea, bacterial infectious diarrhea, fungal infectious diarrhea, and parasitic infectious diarrhea;
and/or the non-infectious diarrhea comprises one or more of non-infectious inflammatory diarrhea, neoplastic diarrhea, malabsorption diarrhea, motility-associated diarrhea, intestinal dysbacteriosis-associated diarrhea, and drug-associated diarrhea;
further preferably, the viral infectious diarrhea comprises one or more of norovirus, rotavirus, sapovirus, astrovirus, adenovirus, enterovirus, and coronavirus;
preferably, the viral diarrhea is diarrhea caused by infection with norovirus or rotavirus;
the bacterial infection comprises one or more of *Salmonella, Shigella, Campylobacter, Yersinia enterocolitica, Vibrio cholerae,* enterotoxigenic *Escherichia coli, Staphylococcus aureus,* and diarrheagenic *Escherichia coli;*
the fungal infection comprises one or more of *Candida, Mucor,* and *Aspergillus;*
the parasitic infection comprises one or more of *Giardia lamblia* (*Giardia*)*, Entamoeba histolytica, Cryptosporidium cuniculus, Cyclospora, Blastocystis hominis, Trichinella,* and *Schistosoma*;
the malabsorption diarrhea is secondary small intestinal malabsorption diarrhea; further preferably, the secondary small intestinal malabsorption diarrhea is dyspeptic diarrhea; still further preferably, the dyspeptic diarrhea is lactose malabsorption diarrhea;
the intestinal dysbacteriosis-associated diarrhea is *Clostridium difficile* dysregulation-associated diarrhea;
the drug in the drug-associated diarrhea is one or more of a laxative (phenolphthalein, senna leaf, castor oil, or the like), a hyperosmotic drug (magnesium sulfate, sodium sulfate, or the like), an antibiotic (penicillins, cephalosporins, lincomycin, clindamycin, or the like), a chemotherapeutic drug (epirubicin, docetaxel, fluorouracil, hydroxycamptothecin, or the like), an antihypertensive drug (propranolol, reserpine, methyldopa, or the like), an antiarrhythmic drug (cardiac glycosides, quinidine, or the like), a diuretic drug (furosemide, ethacrynic acid, or the like), and a lipid-lowering drug (clofibrate, cholestyramine, or the like); further preferably, the drug is one or more of a laxative, a hyperosmotic drug, and an antibiotic.

4. The use according to any one of claims 1-3, wherein the *Bacteroides fragilis* is in the form of live bacteria or morphologically intact inactivated bacteria and/or morphologically incomplete inactivated bacteria or a lysate.

5. The use according to claim 4, wherein the *Bacteroides fragilis* is inactivated by any one or more of the methods including dry heat, moist heat, filtration, organic solvents, chemical reagents, ultraviolet or infrared radiation, fermentation, freeze drying, genetic recombination, and genetic modification or engineering.

6. The use according to claim 4, wherein the *Bacteroides fragilis* is available as a *Bacteroides fragilis* live bacteria solution or inactivated bacteria powder; the *Bacteroides fragilis* inactivated bacteria powder is prepared by the steps of fermentation culture, washing with an aqueous sodium chloride solution and centrifugation, addition of an excipient for resuspension, inactivation, and drying; preferably, the *Bacteroides fragilis* inactivated bacteria powder is prepared by the following method, which comprises the following steps:
(1) fermentation culturing of *Bacteroides fragilis*;
(2) post-fermentation, centrifuging the fermentation liquid to collect bacterial cells, then washing these cells with an aqueous sodium chloride solution at a weight-to-volume ratio of 1 g:(10-30) mL, followed by centrifugation to obtain the washed bacterial cells;
(3) adding a first excipient solution to the washed bacterial cells to mix and resuspend forming a bacterial cell solution, then performing inactivation treatment and centrifugation to collect an inactivated bacterial sludge;
(4) adding a second excipient solution to the inactivated bacterial sludge obtained in step (3) to obtain an inactivated bacteria stock solution; and
(5) drying the inactivated bacteria stock solution obtained in step (4) until a residual water content is less than 5 wt% , thus obtaining the *Bacteroides fragilis* inactivated bacteria powder;
according to an embodiment of the present invention, in step (2), a bacterial count in the fermentation liquid reaches no less than 10⁸ CFU/mL;
according to an embodiment of the present invention, in step (2), a mass concentration of the aqueous sodium chloride solution ranges from 0.6-1.5 wt%, preferably from 0.65-1.2 wt%, more preferably from 0.8-1.0 wt%, most preferably from 0.85-0.95 wt%, such as 0.9 wt%;
according to an embodiment of the present invention, the excipient comprises at least one of mannitol, sorbitol, maltodextrin, lactose, sodium chloride, maltose, sucrose, glucose, trehalose, dextran, proline, lysine, alanine, casein, and skim milk;
according to an embodiment of the present invention, in step (3), a weight-to-volume ratio of the bacterial cells to the first excipient solution is 1 g:(5-40) mL;
according to an embodiment of the present invention, in step (3), the excipient in the first excipient solution has a mass fraction of 4-30 wt%, with the excipient defined as previously described; according to an embodiment of the present invention, in step (3), the solvent of the first excipient solution is selected from an aqueous sodium chloride solution, which are defined as previously described; further preferably, the solvent of the first excipient solution is selected from normal saline, such as a 0.9 wt% aqueous sodium chloride solution;
according to an embodiment of the present invention, in step (3), the inactivation treatment is performed by at least one selected from heat inactivation, freeze inactivation, and chemical inactivation, preferably heat inactivation;
illustratively, the heat inactivation is performed at a temperature of 60-100 °C for a period of 10-60 min;
according to an embodiment of the present invention, in step (4), the second excipient solution is added to allow the total weight of the inactivated bacteria stock solution to be consistent with the weight of the bacterial cell solution before the inactivation in step (3); preferably, the second excipient solution is the same as or different from the first excipient solution;
according to an embodiment of the present invention, the excipient in the second excipient solution has a mass fraction of 4-30 wt%, with the excipient defined as previously described;
preferably, the solvent of the second excipient solution is selected from an aqueous sodium chloride solution, which are as defined previously; further preferably, the solvent of the first excipient solution is selected from normal saline, such as a 0.9 wt% aqueous sodium chloride solution;
illustratively, the second excipient solution is identical to the first excipient solution;
according to an embodiment of the present invention, in step (5), the drying is performed by a method selected from vacuum freeze drying and/or spray drying, preferably vacuum freeze drying; illustratively, the vacuum freeze drying is performed under conditions comprising: a freezing temperature of -20 to -40 °C, a freezing time of 1-3 h, and a vacuum degree of 0.20-0.25 mbar; illustratively, the vacuum freeze drying is performed by a process comprising: pre-freezing at - 40±2 °C for 1-3 h, then pre-freezing at -20±2 °C for 0.5-1 h, finally pre-freezing at -40±2 °C for another 0.5-2 h, and performing primary drying and desorption drying at a vacuum degree of 0.25 mbar to prepare the inactivated bacteria powder;
according to an embodiment of the present invention, in the preparation method, the centrifugation conditions are not specifically limited, provided that the desired centrifugation effect can be achieved;
for example, the centrifugation is performed at a rotation speed of 10000-20000 rpm.

7. The use according to any one of claims 1-6, wherein the composition can be any one of a pharmaceutical composition, food, a healthcare product, or a food additive.

8. The use according to claim 7, wherein the pharmaceutical composition comprises a pharmaceutically effective dose of the *Bacteroides fragilis* inactivated bacteria powder according to claim 6.

9. The use according to claim 8, wherein the pharmaceutical composition is in the dosage form of a pill, a tablet, a granule, a capsule, a powder, a suspension, an oral liquid, or an enema agent; preferably, the pharmaceutical composition is administered orally or by enema;
preferably, the administration cycle of the pharmaceutical composition is intermittent administration, periodic administration, continuous administration, or chronic administration.
